(19) Europäisches Patentamt
European Patent Office
Office européen des brevets

(11) Publication number: 0 055 990 B1

# (12) EUROPEAN PATENT SPECIFICATION

(45) Date of publication of patent specification: 11.06.86

(51) Int. Cl.[4]: C 07 D 487/04, A 61 K 31/40 // (C07D487/04, 209:00, 205:00)

(21) Application number: 82100683.0

(22) Date of filing: 04.08.80

(60) Publication number of the earlier application in accordance with Art. 76 EPC: 0 024 832

## (54) Beta-lactam antibiotics, their preparation and use.

(30) Priority: 10.08.79 GB 7927901
17.04.80 GB 8012724

(43) Date of publication of application:
14.07.82 Bulletin 82/28

(45) Publication of the grant of the patent:
11.06.86 Bulletin 86/24

(84) Designated Contracting States:
BE CH DE FR GB IT LI LU NL SE

(58) References cited:
EP-A-0 001 627
EP-A-0 017 992

(73) Proprietor: BEECHAM GROUP PLC
Beecham House Great West Road
Brentford Middlesex TW8 9BD (GB)

(72) Inventor: Corbett, David Francis
72E Holmesdale Road
Reigate Surrey (GB)

(74) Representative: Hesketh, Alan, Dr. et al
European Patent Attorney
Beecham Pharmaceuticals
Great Burgh Yew Tree Bottom Road
Epsom, Surrey, KT18 5XQ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates the preparation of carbapenem derivatives, and to certain novel antibacterial carbapenem derivatives and compositions containing them.

European Patent Application Publication Number 0001628 discloses a group of synthetic antibacterial agents containing a 7-oxo-1-azabicyclo[3.2.0.]hept-2-ene ring system. EP—A—1627 discloses a similar group of 7-oxo-1-azabicyclo[3.2.0.]hept-2-ene ring systems having a 1-hydroxyethyl substituent at C-6. However all compounds described in those specifications are racemic at C-5 and can only be prepared by a very long synthetic sequence. A new process has been discovered that enables new antibacterial agents to be prepared via the intermediacy of a thiol. These new antibacterial agents can be prepared by a relatively short reaction sequence from natural products and are produced as a desirable single optical isomer at C-5.

Various 7-oxo-1-azabicyclo[3.2.0.]hept-2-ene-2-carboxylic acid derivatives are also disclosed in EP—A—0008514, EP—A—0008888, EP—A—0011173 and EP—A—0017992, which are prior art documents not published before the priority date of the present invention.

The present invention provides a process for the preparation of a compound of formula (I):

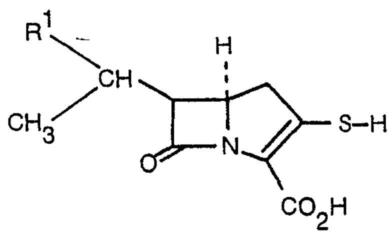

and salts and esters thereof,
wherein

the configuration at the 6-position may be R or S,

$R^1$ is hydrogen, OH, $OSO_3H$ or a salt or a $(C_{1-4})$alkyl ester thereof, $OR^2$, $SR^3$, $OCOR^2$, $OCO_2R^3$ or $OCONHR^3$,

$R^2$ is a $(C_{1-6})$alkyl group, a benzyl group, or a benzyl group substituted on the phenyl ring by $(C_{1-3})$alkoxy, fluorine, bromine, chlorine, or nitro, and

$R^3$ is a $(C_{1-6})$alkyl group, a benzyl group, a phenyl group, a benzyl group substituted on the phenyl ring by $(C_{1-3})$alkoxy, fluorine, bromine, chlorine, or nitro, or a phenyl group substituted by $(C_{1-3})$alkyl, $(C_{1-3})$alkoxy, fluorine, bromine, chlorine, or nitro,

with the proviso that, when $R^1$ is $OSO_3H$ or a salt or $(C_{1-4})$alkyl ester thereof, the C-5 and C-6 hydrogen atoms are *cis*;

which process comprises the reaction of a cleavable ester of a compound of the formula (II):

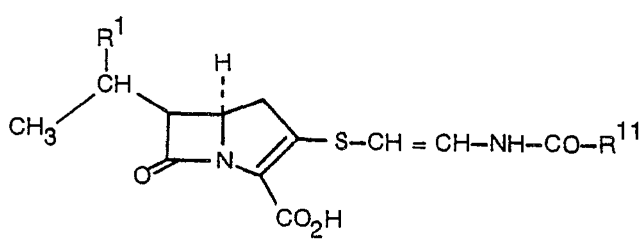

(II)

wherein $R^{11}$ is a methyl or ethyl group;

with the proviso that, when $R^{11}$ is an ethyl group, $R^1$ is $OSO_3H$ or a salt or $(C_{1-4})$alkyl ester thereof;

with a source of hypohalous acid, and optionally thereafter:

i) converting a cleavable ester to a free acid or salt,

ii) converting a cleavable ester to a different cleavable ester.

The present invention also provides a compound of the formula (IA):

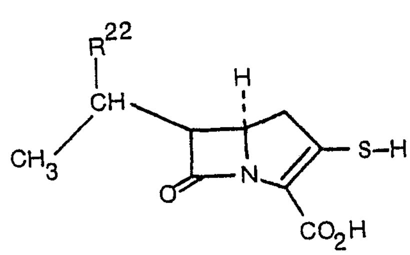

(IA)

and salts and esters thereof,
wherein

the configuration at the 6-position may be R or S,

$R^{22}$ is hydrogen, $OSO_3H$ or a salt or a $(C_{1-4})$alkyl ester thereof, $SR^3$ or $OCONHR^3$, and
$R^3$ is defined as above;
with the proviso that, when $R^{22}$ is $OSO_3H$ or a salt or $(C_{1-4})$alkyl ester thereof, the C-5 and C-6 hydrogen atoms are *cis*.

A group of compounds of interest are those of the formula (IA) wherein $R^{22}$ is hydrogen, $OSO_3H$ or a pharmaceutically acceptable salt or a methyl or ethyl ester thereof, $SR^3$, or $OCONHR^3$ where $R^3$ is a $(C_{1-4})$alkyl, benzyl, phenyl or *p*-nitrobenzyl group.

Suitable values for $R^1$ include hydrogen, hydroxy, $OSO_3H$ or a salt or methyl or ethyl ester thereof, methylthio, ethylthio, phenylthio, benzylthio, *p*-nitrobenzylthio, acetoxy, propionoxy, benzyloxy and phenylacetyloxy.

Preferred values for $R^1$ include hydrogen, hydroxy, acetoxy and $OSO_3H$ or a salt or methyl or ethyl ester thereof.

Although compounds of the formula (I) or (IA) and salts and esters thereof have antibacterial activity, they are primarily envisaged as starting-materials in the processes hereinafter defined.

Hereinafter, all references to formula (I) include formula (IA) and all references to $R^1$ include $R^{22}$, unless the context obviously requires otherwise.

Suitably for use as an intermediate in such processes any sulphate moiety in the group $R^1$ in the compounds of the formula (I) is in the form of a quaternary ammonium salt, for example the benzyldimethyl-n-hexadecylammonium salt, or is in the form of a methyl or ethyl sulphate ester.

Suitably for use as an intermediate the compound of the formula (I) is in the form of a cleavable ester at the C-2 carboxyl. Apt cleavable esters include those cleavable by chemical methods such as hydrogenolysis or hydrolysis or by biological methods.

Suitably the carboxylic acid is esterified by a group of the sub-formula (a), (b), (c) or (d):

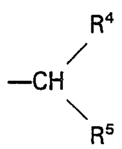 (a) 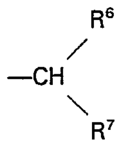 (b) 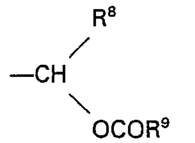 (c) 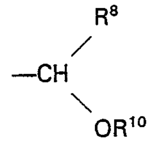 (d)

wherein $R^4$ is a hydrogen atom or an alkyl, alkenyl or alkynyl group of up to 3 carbon atoms; $R^5$ is a hydrogen atom or a methyl group; $R^6$ is a phenyl group or a phenyl group substituted by a fluorine, chlorine or bromine atom or a nitro, methyl or methoxy group; $R^7$ is a hydrogen atom or a phenyl group or a phenyl group substituted by a fluorine, chlorine or bromine atom or a nitro, methyl or methoxy group; $R^8$ is a hydrogen atom or a methyl group; $R^9$ is a $(C_{1-4})$alkyl, phenyl or $(C_{1-4})$alkoxy group or $R^8$ is joined to $R^9$ to form a phthalidyl, dimethylphthalidyl or dimethoxyphthalidyl group; and $R^{10}$ is a $(C_{1-4})$alkyl, phenyl, chlorophenyl or nitrophenyl group; or $CHR^4R^5$ is a phenacyl or bromophenacyl group.

Favourably $R^4$ is a hydrogen atom or a methyl, ethyl, vinyl or ethenyl group. Favourably $R^5$ is a hydrogen atom. Favourably $R^6$ is a phenyl, *p*-bromophenyl, *p*-methoxyphenyl or *p*-nitrophenyl group. Favourably $R^7$ is a hydrogen atom. Favourably $R^9$ is a methyl, t-butyl or ethoxy group or is joined to $R^8$. Favourably $R^{10}$ is a methyl group.

Preferred groups of the sub-formula a) include the methyl and ethyl groups.

Preferred groups of the sub-formula b) include the benzyl and *p*-nitrobenzyl groups.

Preferred groups of the sub-formula c) include the acetoxymethyl, pivaloxyloxymethyl, α-ethoxy-carbonyloxymethyl and phthalidyl groups.

A preferred group of the sub-formula d) is the methoxymethyl group.

Particularly preferred esterifying groups are the *p*-nitrobenzyl and phthalidyl groups.

When the compounds of the formula (I) and salts and esters thereof are intended for use as antibacterial agents, then suitably the compound is in the form of an *in-vivo* hydrolysable ester or pharmaceutically acceptable salt. Suitable *in-vivo* hydrolysable esters include those of sub-formula (c) as hereinbefore defined. Suitable pharmaceutically acceptable salts include those of the alkali and alkaline earth metals; of these the sodium and potassium salts are preferred. These pharmaceutically acceptable salts may be formed at the C-2 carboxyl, and/or at a C-8 sulphate moiety (if present). Thus compounds of the formula (I) wherein $R^1$ is a $OSO_3H$ group or pharmaceutically acceptable salt thereof may be in the form of a di-salt such as the di-sodium salt or di-potassium salt, or may be in the form of a mono-salt of an *in-vivo* hydrolysable ester, or may be in the form of a mono-salt of an acid or may be in the form of a di-acid.

The compounds of the formula (I) may have the *cis*- or *trans*- geometry about the β-lactam, that is to say they have the (5R,6R) or (5R,6S) configuration. Alternatively the compounds of the formula (I) may be presented in the form of a *cis/trans* mixture.

The compounds of the formula (I) may have R or S stereochemistry at C-8 (except of course when $R^1$ is hydrogen) or may be in the form of mixtures thereof.

The compounds of the formula (I) wherein $R^1$ is $OSO_3H$ or a salt or $(C_{1-4})$ ester thereof are suitably in the 8S-configuration as the necessary intermediates are more readily available.

According to the process of the invention, a compound of the formula (I) or salt or ester thereof is prepared from a starting material, which is a cleavable ester of a compound of the formula (II) defined above.

Preferably $R^{11}$ is a methyl group as the intermediates are more readily available.

Suitably the reaction is performed at a non-extreme temperature such as −15°C to +25°C, preferably ambient. Solvents suitable in this reaction are inert organic solvents optionally in the presence of moisture, for example moist acetone or dioxan.

Preferred esters for use in this process are those described hereinbefore as preferred esters for compounds of the formula (I). A particularly preferred ester for use in this process is the *p*-nitrobenzyl ester.

Suitably the hypohalous acid is hypobromous acid or hypochlorous acid; of these hypobromous acid is preferred. Suitable sources of hypohalous acids include N-bromoacetamide, N-chloroacetamide and N-bromopropionamide.

Compounds of the formula (II) may be prepared by the methods of European Patent Application Publication Numbers 0005348, 0005349, 0007152, 0008497, 0043197, Belgian Patent Number 864570, and U.K. Patent Number 1489235.

Compounds of the formula (IA) are also useful for the preparation of compounds of the formula (VIII):

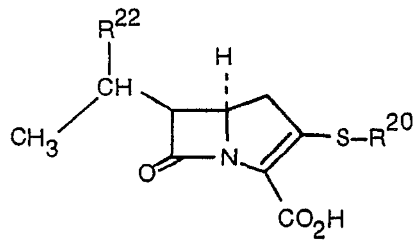

(VIII)

and pharmaceutically acceptable salts and *in-vivo* hydrolysable esters thereof,
wherein

the configuration at the 6-position may be R or S,

$R^{22}$ is defined as above, with the proviso that, if $R^{22}$ is a salt of $OSO_3H$, it should be a pharmaceutically acceptable salt, and

$R^{20}$ is $(C_{1-6})$alkyl, $(C_{3-6})$alkenyl, $(C_{3-6})$alkynyl, aralkyl, $(C_{1-6})$alkanoyl, aralkanoyl, aryloxyalkanoyl or arylcarbonyl, any of such groups being optionally substituted, and the multiple bond in the said alkenyl and alkynyl groups not being present at the carbon atom adjacent to the sulphur atom;

with the proviso that, when $R^1$ is $OSO_3H$ or a pharmaceutically acceptable salt or $(C_{1-4})$ alkyl ester thereof, the C-5 and C-6 hydrogen atoms are *cis*;

by reacting a cleavable ester of a compound of the formula (IA) with a compound of the formula (IX):

$$X—R^{20} \qquad (IX)$$

wherein X is a leaving group,
in the presence of an acid acceptor; and subsequently:

i) converting any cleavable ester group which is not *in-vivo* hydrolysable into a free acid, a pharmaceutically acceptable salt, or an *in-vivo* hydrolysable ester group;

ii) optionally converting any cleavable ester group which is *in-vivo* hydrolysable into a free acid, a pharmaceutically acceptable salt or a different *in-vivo* hydrolysable ester group.

When $R^{20}$ is a $(C_{1-6})$alkyl group suitable substituents include amino, hydroxy, $(C_{1-6})$alkanoyloxy, $(C_{1-6})$alkoxy, benzoyl, $(C_{1-6})$alkanoyl, or carboxy or an ester or pharmaceutically acceptable salt thereof.

When $R^{20}$ is a $(C_{3-6})$alkenyl group wherein the double bond is not present on the carbon adjacent to the sulphur atom, suitable substituents include carboxy or an ester or pharmaceutically acceptable salt thereof, hydroxy or $(C_{1-6})$alkoxy.

When used herein the term "aralkyl" means a $(C_{1-6})$alkyl group substituted by an aryl group, examples of such aryl groups being naphthyl, pyrrolyl, furyl, thienyl, indolyl, thionaphthyl, benzofuryl, imidazoyl, thiazolyl, or any of such groups substituted by one or more groups selected from $(C_{1-3})$alkyl, phenyl, nitro and amino; or a phenyl group optionally substituted by a halogen atom or a $(C_{1-3})$alkoxy, nitro or acetamido group.

A group of compounds of interest is that of the formula (IV):

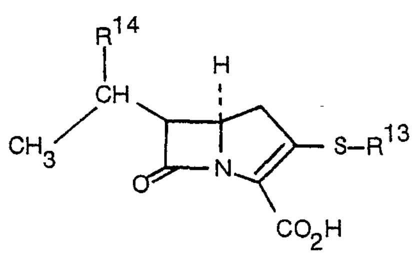

(IV)

and pharmaceutically acceptable salts and in-vivo hydrolysable esters thereof wherein $R^{14}$ is hydrogen, $OSO_3H$ or a pharmaceutically acceptable salt or a methyl or ethyl ester thereof, $SR^3$, or $OCONHR^3$ where $R^3$ is a $(C_{1-4})$alkyl, benzyl, phenyl or *p*-nitrobenzyl group; and $R^{13}$ is $(C_{1-4})$alkyl; $(C_{1-4})$alkyl substituted by a phenyl group optionally substituted by fluorine, chlorine, bromine, methoxy, nitro, amino, acetamido or *p*-nitrobenzyloxycarbonylamino; $(C_{2-4})$alkyl substituted on other than the α-carbon atom by amino, *p*-nitrobenzyloxycarbonylamino, hydroxy, *p*-nitrobenzyloxycarbonyloxy, methoxy, acetoxy or $(C_{1-4})$alkyloxycarbonyl group; $(C_{1-4})$alkanoyl; benzoyl; phenylacetyl; or phenoxyacetyl group.

A similar group of compounds of interest is that of the formula (V):

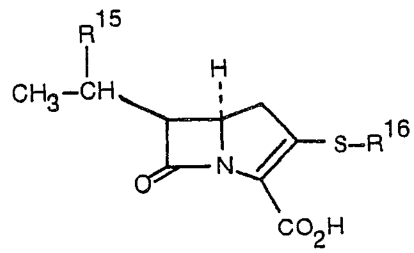

(V)

and pharmaceutically acceptable salts and *in-vivo* hydrolysable esters thereof wherein either:

$R^{15}$ is hydrogen, and $R^{16}$ is $(C_{1-6})$alkyl substituted by amino, benzoyl, $(C_{1-6})$alkanoyl or carboxy or an ester or pharmaceutically acceptable salt thereof, or $R^{16}$ is $(C_{3-6})$alkenyl or $(C_{3-6})$alkynyl wherein the double or triple bond is not present on the carbon atom adjacent to the sulphur atom, or $R^6$ is aralkyl, $(C_{1-6})$alkanoyl, aralkanoyl, aryloxyalkanoyl or arylcarbonyl, any of such groups being optionally substituted; or

$R^{15}$ is $OSO_3H$ or a salt or $(C_{1-4})$alkyl ester thereof, $SR^3$, or $OCONHR^3$ wherein $R^3$ is as defined in relation to compounds of the formula (I); and $R^{16}$ is a group $R^{20}$ wherein $R^{20}$ is as defined in relation to compounds of the formula (III);

with the proviso that when $R^{16}$ is 2-aminoethyl, $R^{15}$ must be $SR^3$ or $OSO_3H$ or a pharmaceutically acceptable salt or $(C_{1-4})$alkyl ester thereof; and with the further proviso that when $R^{15}$ is $OSO_3H$ or a pharmaceutically acceptable salt or $(C_{1-4})$ alkyl ester thereof the C-5 and C-6 protons are *cis*.

Suitably $R^{22}$ in the compounds of the formula (VIII) is hydrogen or, especially, $OSO_3H$ or a pharmaceutically acceptable salt thereof, such as the sodium or potassium salt.

When $R^{20}$ is an alkyl group or substituted alkyl group suitably such alkyl groups contain up to 4 carbon atoms, for example $R^{20}$ aptly may be methyl, ethyl, propyl, butyl, aminomethyl, aminoethyl, aminopropyl, aminobutyl, hydroxyethyl, hydroxypropyl, hydroxybutyl, methoxymethyl, methoxyethyl, ethoxymethyl, ethoxyethyl, propoxypropyl, methoxypropyl, methoxybutyl, acetoxymethyl, acetoxyethyl, propionoxymethyl, propionoxyethyl, phenacyl, acetylmethyl, acetylethyl, propionylmethyl, propionylethyl, carboxymethyl or pharmaceutically acceptable salt thereof, methoxycarbonylmethyl, ethoxycarbonylmethyl, methoxycarbonylethyl, methoxycarbonylpropyl, methoxycarbonylbutyl, ethoxycarbonylethyl, carboxyethyl or a pharmaceutically acceptable salt thereof, carboxypropyl or pharmaceutically acceptable salt thereof, carboxybutyl or pharmaceutically acceptable salt thereof, carboxybutyl or pharmaceutically acceptable salt thereof, and structural isomers thereof; of these, preferred values include methyl, ethyl, propyl, butyl and 2-aminoethyl.

When $R^{20}$ is an aralkyl group more suitably the alkyl moiety is a methylene or ethylene divalent radical. Suitable examples of the aryl moiety are phenyl optionally substituted by one or more substituents selected from halogen, $(C_{1-3})$alkoxy, nitro or acetamido; pyrrolyl optionally substituted by phenyl or $(C_{1-3})$alkyl; thienyl optionally substituted by phenyl or $(C_{1-3})$alkyl; furyl optionally substituted by phenyl or $(C_{1-3})$alkyl; imidazolyl optionally substituted by one or more groups selected from phenyl, nitro, amino, $(C_{1-3})$alkyl; and thiazolyl optionally substituted by one or more groups selected from phenyl, nitro, amino and $(C_{1-3})$alkyl.

More suitably $R^{20}$ is benzyl, bromobenzyl, chlorobenzyl, fluorobenzyl, methoxybenzyl, nitrobenzyl, acetamidobenzyl, thiazolylmethyl, aminothiazolylmethyl, nitrothiazolylmethyl or phenylthizolylmethyl.

Suitably also $R^{20}$ is phenethyl, pyrrolylethyl or optionally substituted imidazolyethyl. In a suitable aspect the imidazolyl ring may be substituted at the C-2 position (that is the carbon atom α to the two nitrogen atoms) by $(C_{1-3})$alkyl or phenyl. In another aspect the imidazolyl ring may be further substituted at the C-4 position or the C-5 position by $(C_{1-3})$alkyl, phenyl, nitro or amino; preferably such substituents are at the C-4 position, and the C-5 position is unsubstituted; alternatively such substituents are at the C-5 position, and the C-4 position is unsubstituted.

In a further aspect $R^{20}$ is $(C_{1-6})$alkanoyl, aralkanoyl, aryloxyalkanoyl or arylcarbonyl, for example acetyl, phenylacetyl, phenoxyacetyl or benzoyl. Of these, acetyl is preferred.

The groups specified above for the C-3 substituent $R^{20}$ are also, where applicable, suitable groups for $R^{13}$ and $R^{16}$.

The groups specified above for the C-6 substituent $R^1$ are also, where applicable, suitable groups for $R^{22}$, $R^{14}$ and $R^{15}$.

Thus it is to be realised that preferred compounds of the formula (VIII) include those of the formula (VI):

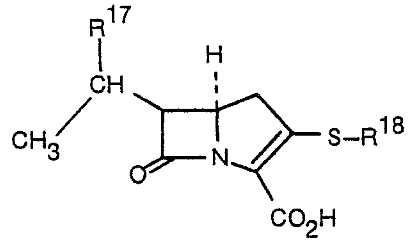

(VI)

or pharmaceutically acceptable salts or *in-vivo* hydrolysable esters thereof, wherein $R^{17}$ is $OSO_3H$ or a pharmaceutically acceptable salt or $(C_{1-4})$alkyl ester thereof, and $R^{18}$ is an optionally substituted $(C_{1-6})$ alkyl group.

Suitable and preferred *in-vivo* hydrolysable ester groups for esterifying the C-2 carboxyl of the compounds of the formulae (VIII) and (IV)—(VI) are those described in relation to compounds of the formula (I). Suitable pharmaceutically acceptable salts of the compounds of the formulae (VIII) and (IV)—(VI) include those of the alkali and alkaline earth metals; of these, the sodium and potassium salts are preferred. These pharmaceutically acceptable salts may be formed at the C-2 carboxyl, and/or at a C-8 sulphate moiety (if present). Thus compounds of the formulae (VIII) and (IV)—(VI) wherein the C—6 substituent contains a $OSO_3H$ group or pharmaceutically acceptable salt thereof may be in the form of a di-salt such as the di-sodium or di-potassium salt, or may be in the form of a mono-salt of an *in-vivo* hydrolysable ester, or may be in the form of a di-acid.

When the thio side-chain at the C-3 position contains an amino group it is preferred that the compounds of the formulae (VIII) and (IV)—(VI) are zwitterionic. The compounds of the formulae (VIII) and (IV)—(VI) may have *cis-* or *trans-* geometry about the β-lactam, that is to say they have the (5R,6R) or (5R,6S) configuration. Alternatively the compounds of the formulae (VIII) and (IV)—(VI) may be presented in the form of a *cis/trans* mixture.

The compounds of the formulae (VIII) and (IV)—(VI) may have R or S stereochemistry at C-8 (except of course when the C-6 substituent is ethyl) or may be in the form of mixtures thereof. The compounds of the formulae (VIII) and (IV)—(VI) wherein the C-6 substituent contains a $OSO_3H$ group or pharmaceutically acceptable salt thereof or $(C_{1-4})$alkyl ester thereof are suitably in the 8S-configuration as the necessary intermediates are more readily available.

Suitable acid acceptors for use in the process for preparing the compounds of the formula VIII are carbonates and bicarbonates such as anhydrous potassium carbonate. The reaction is generally carried out in a dry polar solvent such as dimethylformamide. Suitably the reaction is performed at a non-extreme temperature, for example, −30°C to +60°C, more suitably −10°C to +40°C; and preferably at ambient temperature.

Suitably X is a chlorine bromine or iodine atom or is a sulphonate ester moiety such as a toxylate or mesylate; of these values, iodine and chlorine are preferred. In an alternative aspect, when $R^{20}$ is a methyl or ethyl group, the leaving group X may be dimethyl ether or diethyl ether respectively. In other words X is derived from a trimethyloxonium salt or a triethyloxonium salt. Such salts are conveniently presented as their tetrafluoroborates. Such alkylations involving a trimethyloxonium or triethyloxonium salt are preferably performed in a halogenated hydrocarbon solvent, for example dichloromethane or chloroform, at a depressed temperature for example −80°C to 0°C, more suitably −70°C to −30°C.

In the process for the preparation of compounds of the formula (VIII), any amino group present can be conveniently protected in conventional manner, for example as a *p*-nitrobenzyloxy-carbonylamino group. Similarly any hydroxy group present can be conveniently protected in conventional manner, for example as a *p*-nitrobenzyloxycarbonyloxy group.

Methods of removing protecting groups, cleaving any ester moiety, and converting a free acid or salt to a pharmaceutically acceptable salt or ester, are as detailed in European Patent Application Publication Numbers 0004132, 0005348, 0005349 and 0007152, the contents of which are incorporated herein by reference.

In addition 6-ethylidene compounds of the formula (XII):

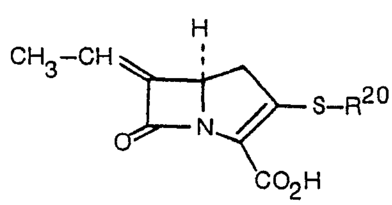

(XII)

and salts and cleavable esters thereof are envisaged as intermediates in the processes mentioned above. The compounds of the formula (XII) may be readily converted to 6-ethyl compounds by known processes such as reduction.

In a particularly preferred aspect the processes of this invention are performed on hydrogenolysable esters, for example the *p*-nitrobenzyl ester, which may be cleaved using an approximately atmospheric pressure of hydrogen at ambient temperature with a transition metal catalyst for example palladium, preferably 5% or 10% palladium on carbon.

The compounds of the formulae (I) and (VIII) and their pharmaceutically acceptable salts and *in-vivo* hydrolysable esters may be employed in the treatment of bacterial infections such as those due to *Staphylococcus aureus*, *Escherichia coli* and *Klebsiella aerogenes*. Thus the present invention provides a pharmaceutical composition which comprises a compound of the formulae (IA) and salts and esters thereof and a pharmaceutically acceptable carrier.

The compositions of this invention may be prepared by conventional methods of preparing antibiotic compositions and in conventional manner may be adapted for oral, topical or parenteral administration.

Aptly, the compositions of this invention are in the form of a unit-dose composition adapted for oral administration.

Alternatively the compositions of this invention are in the form of a unit dose composition adapted for administration by injection.

Unit-dose forms according to this invention will normally contain from 50 to 500 mg of a compound of this invention, for example about 62.5, 100, 125, 150, 200, 250 or 300 mg. Such compositions may be administered from 1 to 6 times a day or more conveniently 2, 3 or 4 times a day so that the total daily dose for a 70 kg adult is about 200 to 2000 mg, for example about 400, 600, 750, 1000 or 1500 mg.

The compositions of this invention may be used to treat infections of the respiratory tract, urinary tract or soft tissues in humans, or mastitis in cattle.

The carriers used in the compositions of this invention may include diluents, binders, disintegrants, lubricants, colours, flavouring agents or preservatives in conventional manner. Thus suitable agents include lactose, starch, sucrose, calcium phosphate, sorbitol, polyvinylpyrrolidone, acacia, gelatin, tragacanth, potato starch or polyvinylpolypyrrolidone, magnesium stearate or sodium lauryl sulphate.

Orally administrable forms of the compositions of this invention are most suitably in the form of unit-dose units such as tablets or capsules.

The present invention also provides synergistic pharmaceutical compositions which comprise a pharmaceutical composition as hereinbefore described which also contains a penicillin or a cephalosporin.

Suitable penicillins for inclusion in the compositions of this invention include benzyl penicillin, phenoxymethylpenicillin, ampicillin or a pro-drug therefor, amoxycillin or a pro-drug therefor, carbenicillin or a pro-drug therefor, ticarcillin or a pro-drug therefor, suncillin, sulbenicillin, azlocillin or mezlocillin.

Particularly suitable penicillins for inclusion in orally administrable compositions of this invention include ampicillin and its orally administrable pro-drugs, amoxycillin and its orally administrable pro-drugs and orally administrable pro-drugs of carbenicillin. Thus particularly suitable penicillins include ampicillin anhydrate, ampicillin trihydrate, sodium ampicillin, talampicillin hydrochloride, pivampicillin hydrochloride and bacampicillin hydrochloride; amoxycillin trihydrate, sodium amoxycillin; and the sodium salts of the phenyl and 5-indanyl α-esters of carbenicillin.

A preferred penicillin for inclusion in the orally administrable compositions of this invention is amoxycillin trihydrate. A further preferred penicillin for inclusion in the orally administrable compositions of this invention is ampicillin trihydrate.

Particularly suitable penicillins for inclusion in injectably administrable compositions of this invention include injectable salts such as the sodium salt of ampicillin, amoxycillin, carbenicillin and ticarcillin.

A preferred penicillin for inclusion in the injectably administrable compositions of this invention is sodium amoxycillin. A further preferred penicillin for inclusion in the injectably administrable compositions of this invention is sodium ampicillin.

Particularly suitable cephalosporins for inclusion in the compositions of this invention include cephaloridine, cephalexin, cephradine, cefazolin and cephalothin.

A particularly suitable cephalosporin for inclusion in the orally administrable compositions of this invention is cephalexin.

Particularly suitable cephalosporins for inclusions in the injectably administrable compositions of this invention include cephaloridine, cefazolin and cephradine, generally as their pharmaceutically acceptable salt.

The weight ratio between compound of this invention and penicillin or cephalosporin is generally from 10:1 to 1:10, more usually from 5:1 to 1:5 and normally from 3:1 to 1:3.

The penicillin or cephalosporin is generally utilised in its conventionally administered amount.

Suitable methods of formulation include those described in the aforementioned European Patent Applications.

The following Examples serve to illustrate the invention.

Example 1

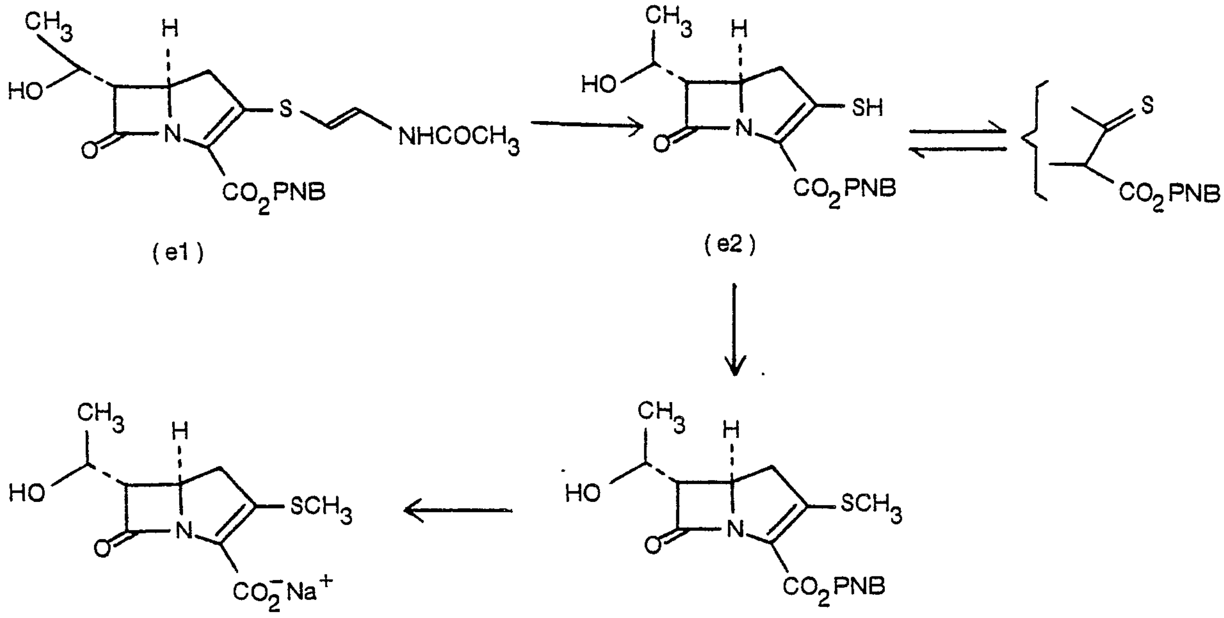

(e1) (e2)

(e10) (e3)

Example 1a

p-Nitrobenzyl (5R,6S)-3-mercapto-6[(S)-1-hydroxyethyl]-7-oxo-1-azabicyclo[3.2.0]hept-2-ene-2-carboxylate

A solution of the ester (e1) (160 mg) in acetone (3 ml) containing water (10 drops) was cooled to −20°. A solution of N-bromoacetamide (55 mg) in acetone (0.5 ml) was added with stirring, and stirring was continued for 20 min at −20°. Chloroform (30 ml) was added and the solution was washed with water (30 ml). The dried ($MgSO_4$) organic layer was concentrated in vacuo to afford a foam (119 mg) which contained the thiol (e2); $\nu_{max.}$ ($CHCl_3$) 1775, 1705 $cm^{-1}$.

Example 1b

p-Nitrobenzyl (5R,6S)-3-methylthio-6-[(S)-1-hydroxyethyl]-7-oxo-1-azabicyclo[3.2.0]hept-2-ene-2-carboxylate

The product from Example 1a was dissolved in DMF (1 ml) and to the solution was added anhydrous potassium carbonate (25 mg) and methyl iodide (0.2 ml). After stirring the mixture vigorously for 20 min, ethyl acetate (30 ml) was added, and the organic layer was washed with water (2 × 30 ml) and brine (30 ml). The solution was dried ($MgSO_4$) and the solvent evaporated in vacuo to leave a residue which was chromatographed on silica using 2% EtOH in $CHCl_3$ to elute. The title methylthio-derivative (e3) was obtained as a white solid (29 mg); $\nu_{max.}$ (KBr) 3450 (br) 1765 and 1695 $cm^{-1}$; $\lambda_{max.}$ (EtOH) 320 (11,900) and 266 nm (11,300); δ (DMF-$d_7$) 1.29 (3H, d, *J* 6.5 Hz, $C\underline{H}_3CH$), 2.45 (3H, s, $CH_3S$), 3.22 and 3.45 (each 1H, dd, *J* 9 and 18 Hz, 4-$CH_2$), 3.50 (1H, dd, J 3 and 4 Hz, 6-CH), *ca.* 4.1 (1H, m, $CH_3C\underline{H}$), 4.25 (1H, dt, *J* 3 and 9 Hz, 5-CH), 5.17 (1H, d, *J* 4.5 Hz, OH), 5.32 and 5.57 (each 1H, d, J 14 Hz, $CH_2CO_2$), 7.82 and 8.27 (each 2H, d, *J* 9 Hz, $C_6\underline{H}_4$—$NO_2$). [$M^+$, 378.0884. $C_{17}H_{18}N_2O_6S$ requires *M*, 378.0882].

Example 1c

Sodium (5R,6S)-3-methylthio-6-[(S)-1-hydroxyethyl]-7-oxo-1-azabicyclo[3.2.0]hept-2-ene-2-carboxylate

5% Pd on C catalyst (60 mg) was shaken with hydrogen in 30% aqueous dioxan (10 ml) at ambient pressure and temperature for 0.5 h. A solution of the ester (e3) in 30% aqueous dioxan (2 ml) was added to the vessel and hydrogenation was continued for a further 3.25 h. Sodium bicarbonate (10 mg) was added and the mixture was filtered through Celite washing the pad well with water (20 ml). The filtrate was concentrated in vacuo to *ca.* 20 ml and the aqueous solution was washed with ethyl acetate (3 × 30 ml), before concentrating in vacuo to a volume of *ca.* 5 ml. The resulting solution was loaded onto a column (15 × 2.5 cm) of Biogel P2 which was eluted with water. Fractions containing the title sodium salt (e10) were identified by the chromophore at $\lambda_{max.}$ ($H_2O$) 302 nm in the UV spectrum. These were collected and combined to afford an aqueous solution of the salt (e10) (*ca.* 11 mg estimated by UV). The salt could be obtained as a hygroscopic solid by removal of the solvent in vacuo (aided by the addition of ethanol and toluene, respectively).

Example 2

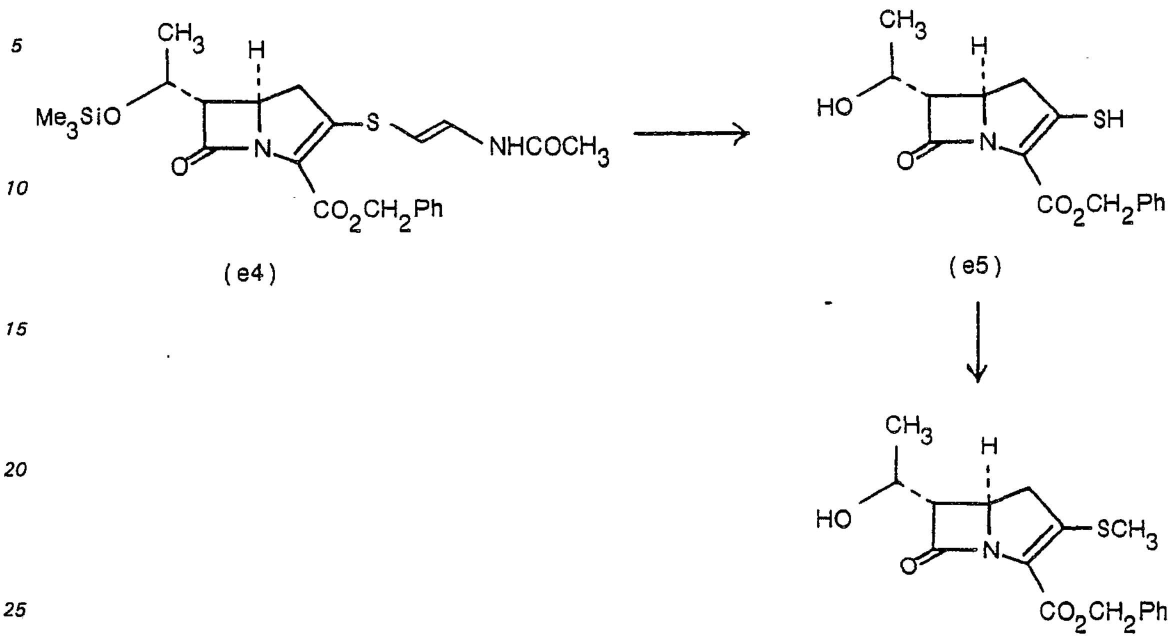

(e4) (e5) (e6)

Example 2a

Benzyl (5R,6S)-3-mercapto-6[(S)-1-hydroxy ethyl]-7-oxo-1-azabicyclo[3.2.0]hept-2-ene-2-carboxylate

A solution of the trimethylsilyl ether (e4) (123 mg) in acetone (3 ml) containing water (3 drops) was treated with N-bromoacetamide (39 mg) at −20° with stirring. After 20 min ethyl acetate (30 ml) was added and the solution was washed with water (30 ml) followed by brine (30 ml), before drying ($MgSO_4$) and concentrating in vacuo. To the residue was added chloroform followed by ether and the solid which precipitated was removed by filtration. The mother liquors were concentrated in vacuo to leave a foam (86 mg) which contained the thiol (e5); $\nu_{max.}$ ($CHCl_3$) 1775 and 1700 $cm^{-1}$.

Example 2b

Benzyl (5R,6S)-3-methylthio-6-[(S)-1-hydroxyethyl]-7-oxo-1-azabicyclo[3.2.0]hept-2-ene-2-carboxylate

The product from Example 2a was dissolved in DMF (1 ml), and anhydrous potassium carbonate (20 mg) followed by methyl iodide (0.5 ml) were added to the solution. After stirring the mixture for 1.5 h, ethyl acetate (30 ml) was added and the organic solution was washed with water (2 × 30 ml) and brine (30 ml). The dried ($MgSO_4$) solution was concentrated in vacuo and the residue chromatographed on silica gel using 20% petroleum ether (60°—80°) in ethyl acetate to elute. The title methylthio-derivative (e6) was obtained as a gum (3 mg); $\nu_{max.}$ ($CHCl_3$) 3400 (br), 1780 and 1700 $cm^{-1}$; $\lambda_{max.}$ (EtOH) 319 nm; δ ($CHCl_3$) 1.36 (3H, d, *J* 6.5 Hz, $\underline{CH_3}CH$), 2.37 (3H, s, $SCH_3$), *ca.* 3.2 (3H, m, 6-CH and 4-$CH_2$), ca. 4.15 (2H, m, 5-C$\underline{H}$ and C$\underline{H}$.$CH_3$), 5.30 (2H, AA'X, $\underline{CH_2}Ph$) and 7.35 (5H, m, $CH_2\underline{Ph}$). [$M^+$, 333.1034. $C_{17}H_{19}NO_4S$ requires *M*, 333.1033].

Example 3

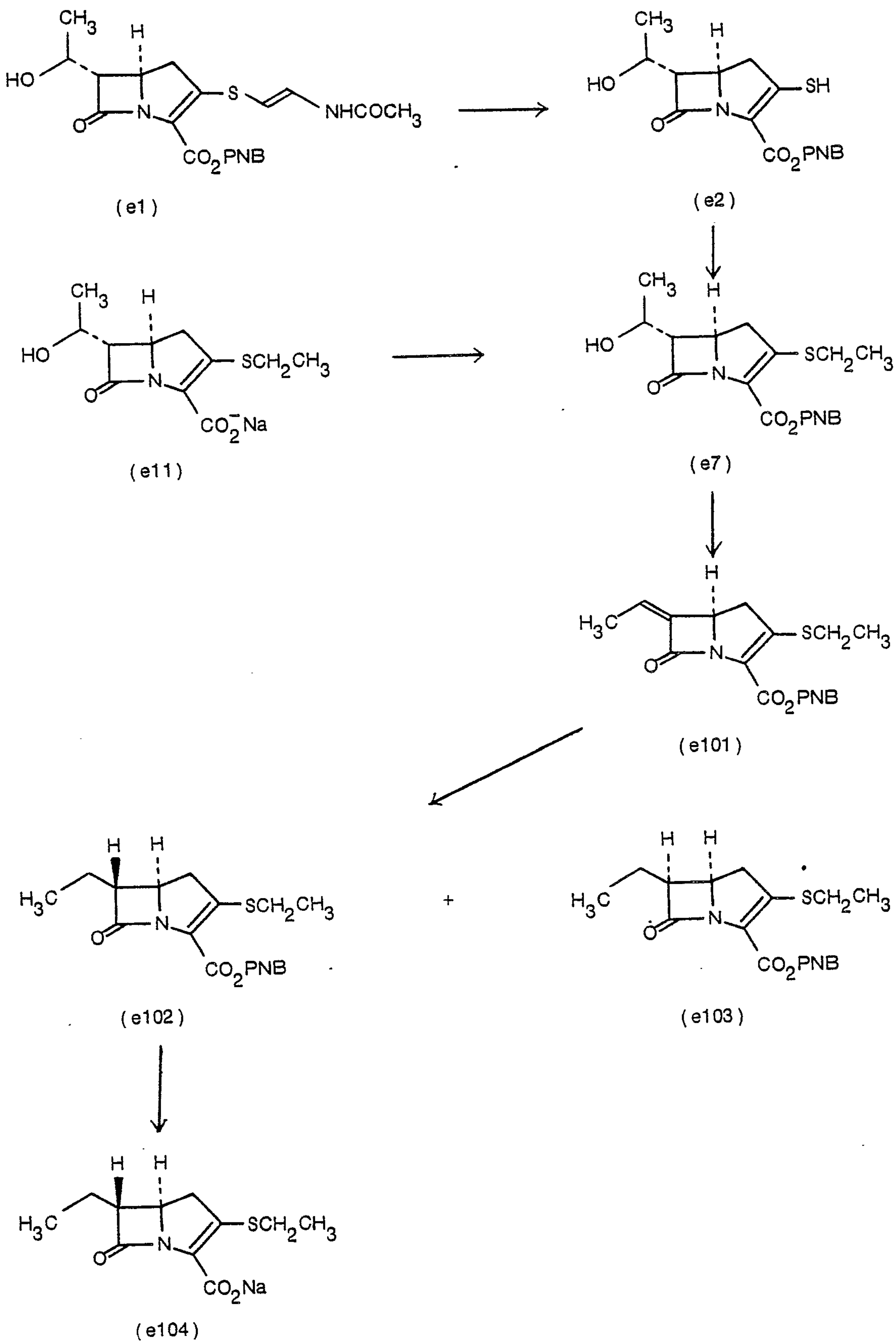

(e1)

(e2)

(e11)

(e7)

(e101)

(e102)

+

(e103)

(e104)

*Method 1*

Example 3a

p-Nitrobenzyl (5R,6S)-3-mercapto-6[(S)-1-hydroxyethyl]-7-oxo-1-azabicyclo[3.2.0]hept-2-ene-2-carboxylate

A solution of the ester (e1) (500 mg) in acetone (10 ml) containing water (1 ml) was treated with N-bromoacetamide (154 mg) with stirring at −20° for 20 min. Work-up as described in Example 1a afforded a foam (519 mg) which contained the thiol (e2).

Example 3b

p-Nitrobenzyl (5R,6S)-3-ethylthio-6-[(S)-1-hydroxyethyl]-7-oxo-1-azabicyclo[3.2.0]hept-2-ene-2-carboxylate

The product from Example 3a was treated with ethyl iodide (0.75 ml) and anhydrous $K_2CO_3$ (250 mg) in DMF (5 ml) in a way analogous to that described in Example 1b. Work-up as also described therein gave a product which was chromatographed on silica using a gradient elution with ethyl acetate/petroleum ether mixtures (from 80% to 100% ethyl acetate). The title ethylthio-derivative was obtained as a white crystalline solid (120 mgs); m.p. 180—183°; $\nu_{max.}$ (KBr) 3490, 1760 and 1700 $cm^{-1}$; $\lambda_{max.}$ (EtOH) 320 (12,600) and 266 nm (11,300). δ (DMF-$d_7$) 1.26 (3H, t, *J* 7.5 Hz, $C\underline{H}_3CH_2$), 1.27 (3H, d, *J* 6.5, $C\underline{H}_3CH$), 2.94 (1H, q, J 7.5 Hz, $SC\underline{H}_2CH_3$), 3.33 (1H, d, *J* 9 Hz, 4-$CH_2$), 3.49 (1H, dd, *J* 3 and 4 Hz, 6-CH), 4.09 (1H, m, $CH_3C\underline{H}$), 4.24 (1H, dt, *J* 3 and 9 Hz, 5-CH), 5.13 (1H, d, *J* 5 Hz, OH), 5.29 and 5.54 (each 1H, d, *J* 14 Hz, $C\underline{H}_2CO_2$), 7.82 and 8.26 (each 2H, d, *J* 8.5 Hz, $C_6\underline{H}_4$—$NO_2$). [$M^+$, 392.1045, $C_{18}H_{20}N_2O_6S$ requires *M*, 392.1040].

*Method 2*

Example 3c

p-Nitrobenzyl (5R,6S)-3-mercapto-6[(S)-1-hydroxyethyl]-7-oxo-1-azabicyclo[3.2.0]hept-2-ene-2-carboxylate

The ester (e1) (200 mg) was converted into the thiol derivative (e2) by the method described in Example 1a.

Example 3d

p-Nitrobenzyl (5R,6S)-3-ethylthio-6-[(S)-hydroxyethyl]-7-oxo-1-azabicyclo[3.2.0]hept-2-ene-2-carboxylate

A solution of the product from Example 3c in dichloromethane (10 ml) was stirred vigorously with anhydrous potassium carbonate (120 mg) and triethyloxonium tetrafluoroborate (84 mg) at −10° for 20 min. The mixture was allowed to warm to room temperature for 10 min, and was then diluted with more methylene chloride (20 ml). The solution was washed with water and dilute brine, then dried ($MgSO_4$) and concentrated in vacuo. The residue was chromatographed on a column of silica gel using 20% petroleum ether in ethyl acetate followed by ethyl acetate to elute.

The ethylthio-derivative (e7), identical in all respects to the product isolated by method 1 (Example 3b), was obtained as a white solid (19 mg).

Example 3e

Sodium (5R,6S)-3-ethylthio-6-[(S)-1-hydroxyethyl]-7-oxo-1-azabicyclo[3.2.0]hept-2-ene-2-carboxylate

Hydrogenolysis of the ethylthio-derivative (e7) (120 mg) was performed in the manner described in Example 1c. (150 mg 5% Pd-C, 26 mg $NaHCO_3$). After Biogel P2 chromatography (column 25 × 2.5 cm) the title salt (e11) (32 mg) was obtained in aqueous solution; $\lambda_{max.}$ 302 nm. Part of the solution was concentrated in vacuo, the final traces of water being removed by azeotroping from ethanol and then toluene, to afford the salt (e11) as a hygroscopic solid; $\nu_{max.}$ (KBr) 1750 and 1590 $cm^{-1}$; δ ($D_2O$) 1.21 (3H, t, *J* 7.5 Hz, $C\underline{H}_3CH_2$), 1.26 (3H, d, *J* 6.5 Hz, $C\underline{H}_3CH$), 2.78 (2H, q, *J* 7.5 Hz, $SC\underline{H}_2CH_3$), 3.14 (2H, m, 4-$CH_2$), 3.39 (1H, dd, *J* 3 and 5.5 Hz, 6-CH), 4.02 (1H, m, 5-CH) and 4.15 (1H, m, $CH_3C\underline{H}$), (reference HOD at δ 4.60).

Example 3f

p-Nitrobenzyl (5R)-3-(ethylthio)-6-Z-ethylidene-7-oxo-1-azabicyclo[3.2.0]hept-2-ene-2-carboxylate

p-Nitrobenzyl (5R,6S)-3-ethylthio-6-[(S)-1-hydroxyethyl]-7-oxo-1-azabicyclo[3.2.0]hept-2-ene-2-carboxylate (e7) (237 mg; 0.60 mM) was dissolved in dry tetrahydrofuran (40 ml) and cooled to 0°C. Triphenylphosphine (634 mg; 2.42 mM) was added with stirring, followed by a solution of diethylazodicarboxylate (421 mg; 2.42 mM) in dry tetrahydrofuran (20 ml). The solution was then allowed to reach room temperature and stirred for a further 20 minutes. The solvent was removed at reduced pressure and the residue partitioned between ethyl acetate and water. The organic layer was washed with an additional volume of water, saturated sodium chloride solution and dried over anhydrous magnesium sulphate. Filtration and removal of the solvent at reduced pressure gave the crude ethylidene as an orange oil. This oil was dissolved in the minimum volume of ethyl acetate/hexane (1:1) and chromatographed over silica gel (20 mg), eluting with a gradient of 50—75% ethyl acetate/hexane. The title ethylidene derivative (e101) was obtained as a pale yellow oil (185 mg). Trituration with diethyl ether/hexane gave a pale yellow solid (130 mg), ν max ($CHBr_3$) 1755, 1700, 1605 $cm^{-1}$.

Example 3g

p-Nitrobenzyl (5R,6R)-3-ethylthio-6-ethyl-7-oxo-1-azabicyclo[3.2.0]hept-2-ene-2-carboxylate (e102) and p-nitrobenzyl (5R,6S)-3-ethylthio-6-ethyl-7-oxo-1-azabicyclo [3.2.0]hept-2-ene-2-carboxylate (e103)

A solution of the ethylidene (e101) (127 mg) in tetrahydrofuran (10 ml) was cooled to −10°C. To this

stirred solution was added a solution of sodium borohydride (52 mg) in 0.05 M pH 7.0 phosphate buffer (2 ml). The reaction mixture was allowed to reach 5°C and stirring continued at this temperature for 1 hour. Ethyl acetate (50 ml) was added and the organic solution washed with water, saturated sodium chloride solution, and dried over anhydrous magnesium sulphate. Filtration and removal of the solvent at reduced pressure gave the crude product as a pale yellow oil. Silica gel column chromatography eluting with 1:1 hexane/ethyl acetate gave a mixture of p-nitrobenzyl (5R,6R)-3-ethylthio-6-ethyl-7-oxo-1-aza-bicyclo[3.2.0]hept-2-ene-2-carboxylate (e102) and p-nitrobenzyl (5R,6S)-3-ethylthio-6-ethyl-7-oxo-1-aza-bicyclo [3.2.0]hept-2-ene-2-carboxylate (e103) (ratio 5:1 by h.p.l.c.) as a colourless oil (14 mg). Crystallisation from ether/hexane gave a white solid, m.pt. 108—115°C, λ max (EtOH) 322 nm, 267 nm, ν max ($CHBr_3$) 1775, 1700, 1605, 1550 $cm^{-1}$, $\delta_H$ ($CDCl_3$) 1.06 (3H, t, $C\underline{H}_3CH_2CH$), 1.32 (3H, t, $C\underline{H}_3$ $CH_2S$), 1.85 (2H, m, $CH_3C\underline{H}_2CH$), 2.85 (2H, q, $SC\underline{H}_2$), 2.7—3.3 (3H, m, 4-$C\underline{H}_2$ + 6-$C\underline{H}$), 3.97 (1H, dt, 5-$C\underline{H}$), 5.37 (2H, q, $C\underline{H}_2Ar$), 7.65 (2H, d, aromatic protons), 8.21 (2H, d, aromatic protons), m/e (relative intensity) 376.1098 (32%; $C_{18}H_{20}N_2O_5S$ requires 376.1091), 347(2), 306(100), 273(20), 255(10), 170(20), 136(35), 126(40).

Example 3h

Sodium (5R,6R)-3-ethylthio-6-ethyl-7-oxo-1-azabicyclo [3.2.0]hept-2-ene-2-carboxylate

5% Palladium on carbon catalyst (15 mg) was shaken with hydrogen in 30% aqueous 1,4-dioxan (5 ml) at ambient pressure and temperature for 20 minutes. A solution of the ester (e102) (10 mg) in 1,4-dioxan (5 ml) was added and hydrogenation was continued for a further 3.5 hours. Sodium bicarbonate (3 mg) was added and the suspension filtered through Celite, washing well with water (25 ml). The filtrate was concentrated at reduced pressure to approximately 15 ml and washed with ethyl acetate (3 × 25 ml). The aqueous solution was further concentrated to about 10 ml and column chromatographed over Biogel P—2, eluting with water. Fractions containing sodium (5R,6R)-3-ethylthio-6-ethyl-7-oxo-1-azabicyclo[3.2.0]hept-2-ene-2-carboxylate (e104) were identified by the chromophore at λ max ($H_2O$) 300 nm in the u.v. spectrum and combined (1.6 mg based on Em 8,000).

Example 4

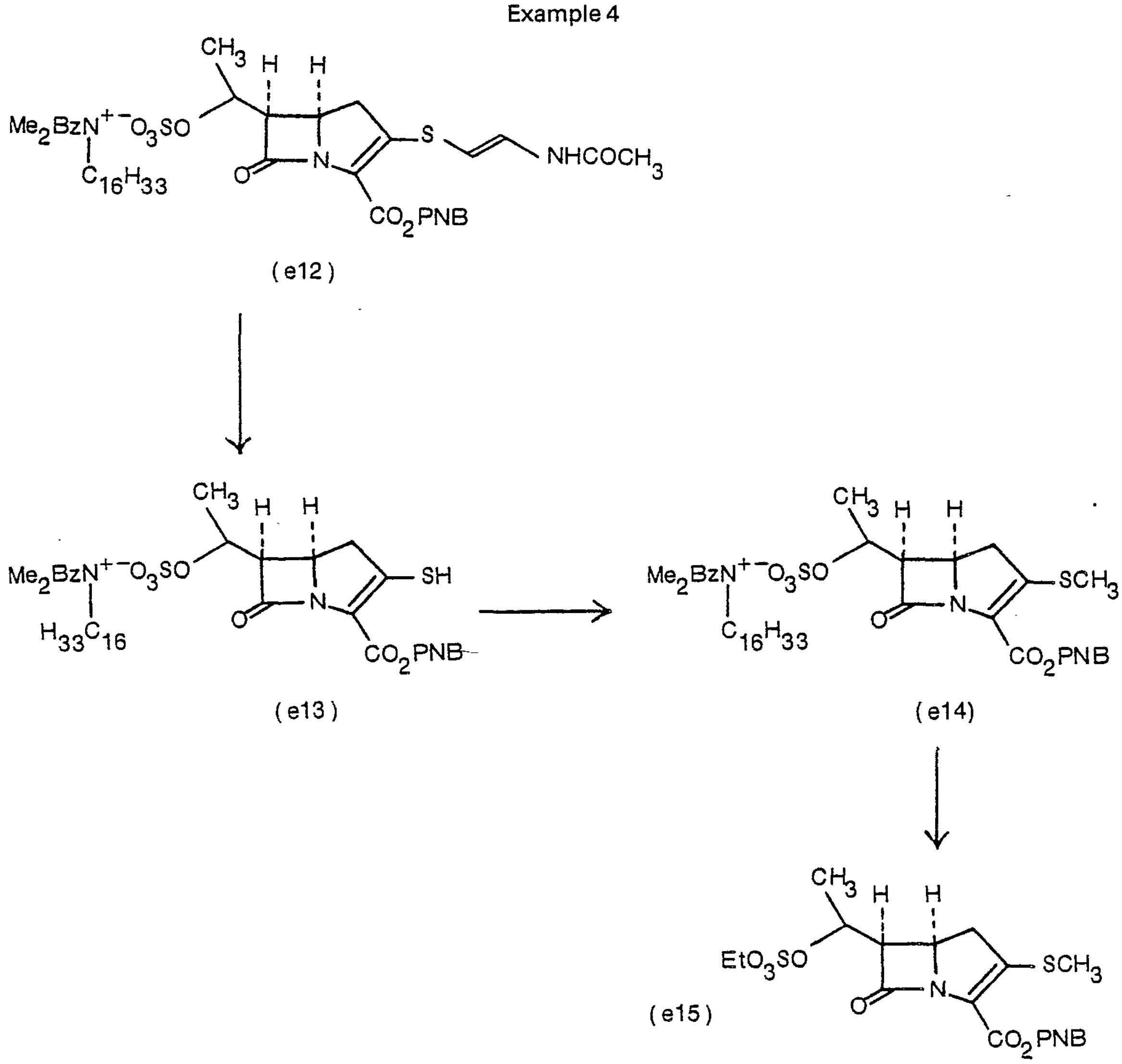

(e12)

(e13)

(e14)

(e15)

### Example 4a

Benzyldimethyl-n-hexadecylammonium salt of p-nitrobenzyl (5R,6R)-3-mercapto-6-[(S)-1-hydroxysulphonyloxyethyl]-7-oxo-1-azabicyclo[3.2.0]hept-2-ene-2-carboxylate

A solution of the quaternary ammonium salt (e12) (409 mg) in 7% aqueous acetone (7.5 ml) was cooled to −20°, and a solution of N-bromoacetamide (70 mg) in acetone (1 ml) was added with stirring. After 20 min at −20° chloroform (50 ml) was added, and the solution was washed with dilute brine (At this stage it was necessary to filter the mixture through Celite in order to break up the emulsion which had formed). The organic layer was dried ($MgSO_4$) and the solvent removed in vacuo to afford a foam (380 mg) which contained the thiol (e13); $\nu_{max.}$ ($CHCl_3$) 1775 and 1700 $cm^{-1}$.

### Example 4b

Benzyldimethyl-n-hexadecylammonium salt of p-nitrobenzyl (5R,6R)-3-methylthio-6-[(S)-1-hydroxysulphonyloxyethyl]-7-oxo-1-azabicyclo[3.2.0]hept-2-ene-2-carboxylate

A solution of the product from Example 4a in DMF (3 ml) was stirred with anhydrous potassium carbonate (75 mg) and methyl iodide (0.3 ml) at room temperature for 20 min. Chloroform (50 ml) was added and the organic solution washed with brine (2 × 50 ml), water (2 × 50 ml) and brine again (50 ml). The dried ($MgSO_4$) solution was evaporated in vacuo and the residue chromatographed on silica gel using a gradient elution of chloroform to 30% ethanol in chloroform. Fractions containing the product (t.l.c.) were combined and concentrated in vacuo to afford the title methylthio-derivative (e14) as a foam (118 mg); $\nu_{max.}$ ($CHCl_3$) 1775 and 1700 $cm^{-1}$; $\lambda_{max.}$ (EtOH) 318 and 268 nm.

### Example 4c

p-Nitrobenzyl (5R,6R)-3-methylthio-6-[(S)-1-ethoxysulphonyloxyethyl]-7-oxo-1-azabicyclo[3.2.0]hept-2-ene-2-carboxylate

A solution of the quaternary ammonium salt (e14) (115 mg) in dichloromethane (10 ml) was stirred with triethyloxonium tetrafluoroborate (30 mg) for 15 min at room temperature. Chloroform (20 ml) was then added and the solution was washed with water (20 ml), dried ($MgSO_4$) and concentrated in vacuo. The product was rapidly chromatographed on silica gel (230—400 mesh ASTM) using 20% petroleum ether in ethyl acetate to elute. The title diester (e15) was obtained as a gum (29 mg); $\nu_{max.}$ ($CHCl_3$) 1780 and 1700 $cm^{-1}$; $\lambda_{max.}$ (EtOH) 317 and 267 nm; δ ($CDCl_3$) 1.42 (3H, t, *J* 7 Hz, $C\underline{H}_3CH_2$), 1.66 (3H, d, *J* 6 Hz, $C\underline{H}_3CH$), 2.41 (3H, s, $SCH_3$), 3.10 (1H, dd, *J* 18 and 10 Hz, 4-$C\underline{H}_aH_b$), 3.36 (1H, dd, *J* 18 and 9 Hz, 4-$CH_a\underline{H}_b$), 3.84 (1H, dd, *J* 6 and 10 Hz, 6-CH), 4.35 (2H, q, *J* 7 Hz, $OC\underline{H}_2CH_3$), 4.37 (1H, m, 5-CH), 5.03 (1H, m, $CH_3C\underline{H}$), 5.20 and 5.48 (each 1H, d, *J* 13.5, $CH_2CO_2$), 7.60 and 8.19 (each 2H, d, *J* 8.5 Hz, $C_6H_4NO_2$).

Example 5

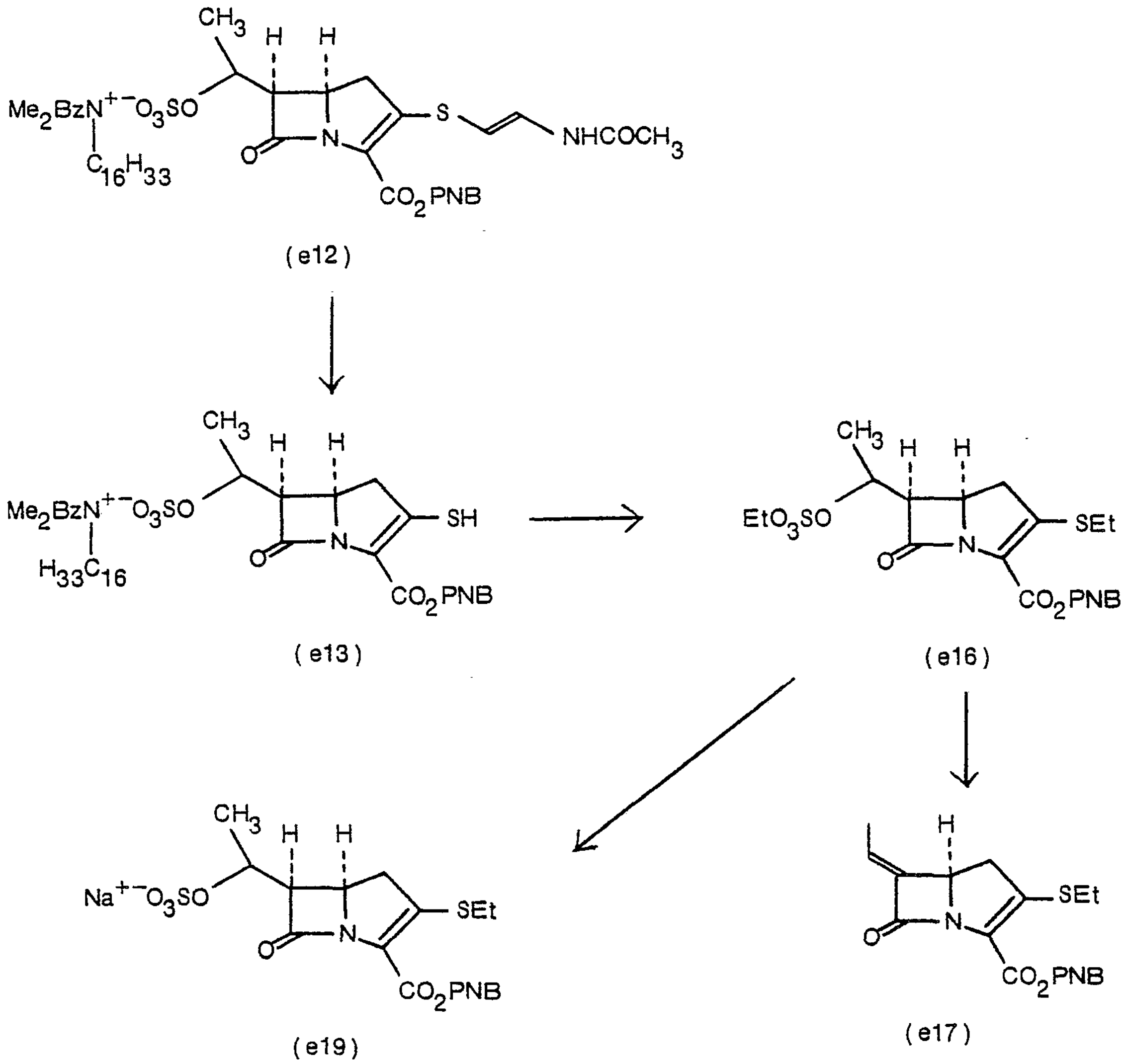

(e12) (e13) (e16) (e19) (e17)

Example 5a

Benzyldimethyl-n-hexadecylammonium salt of p-nitrobenzyl (5R,6R)-3-mercapto-6-[(S)-1-hydroxy-sulphonyloxyethyl]-7-oxo-1-azabicyclo[3.2.0]hept-2-ene-2-carboxylate

The quaternary ammonium salt (e12) (660 mg) was converted into the thiol derivative (e13) by the method described in Example 4a.

Example 5b

p-Nitrobenzyl (5R,6R)-3-ethylthio-6-[(S)-1-ethoxysulphonyloxyethyl]-7-oxo-1-azabicyclo[3.2.0]hept-2-ene-2-carboxylate

The product from Example 4a was dissolved in dry dichloromethane (15 ml) and to the solution was added anhydrous potassium carbonate (205 mg) and Meerwein's reagent (282 mg). The mixture was stirred for 15 min at room temperature and was then diluted with chloroform (30 ml). The organic solution was washed with very dilute brine, dried ($MgSO_4$) and concentrated in vacuo. Rapid chromatography of the residue on silica gel (230—400 ASTM) using 20% petroleum ether/ethyl acetate to elute afforded the title diester as a foam (189 mg); $\nu_{max.}$ ($CHCl_3$) 1780 and 1705 $cm^{-1}$; δ ($CDCl_3$) 1.33 (3H, t, *J* 7 Hz, $C\underline{H}_3CH_2S$), 1.42 (3H, t, *J* 7 Hz, $C\underline{H}_3CH_2O$), 1.66 (3H, d, *J* 6 Hz, $C\underline{H}_3CH$), 2.90 (2H, q, *J* 7 Hz, $CH_3C\underline{H}_2S$), *ca.* 3.25 (2H, m, 4-$CH_2$), 3.84 (1H, dd, *J* 6 and 10 Hz, 6-CH), *ca.* 4.30 (1H, m, 5-CH), 4.35 (2H, q, *J* 7 Hz, $CH_3C\underline{H}_2S$), *ca.* 5.05 (1H, m, $C\underline{H}CH_3$), 5.21 and 5.48 (each 1H, d, *J* 14 Hz, $CH_2CO_2$), 7.61 and 8.20 (each 2H, d, *J* 8.5 Hz, $C_6H_4$—$NO_2$). (The n.m.r. spectrum revealed that some impurity was present in the product).

Example 5c

p-Nitrobenzyl (5R)-3-ethylthio-6-[(E)-ethylidene]-7-oxo-1-azabicyclo[3.2.0]hept-2-ene-2-carboxylate

The diester (e16) (60 mg) was stirred with anhydrous potassium carbonate (41 mg) in DMF (0.5 ml) at room temperature for 30 min. Ethyl acetate (30 ml) was added and the solution was washed with water

(2 × 30 ml) and brine (20 ml) before drying ($MgSO_4$) and evaporating in vacuo. The product was chromatographed on silica gel using 30% petroleum ether in ethyl acetate to elute. The title ethylidene derivative (e17) was isolated as a gum; $\nu_{max.}$ ($CHCl_3$) 1770 and 1705 $cm^{-1}$; δ ($CDCl_3$) 1.32 (3H, t, *J* 7 Hz, $\underline{CH_3}CH_2$), 1.83 (3H, d, *J* 7 Hz, $\underline{CH_3}CH$), 2.84 (2H, q, *J* 7 Hz, $S\underline{CH_2}CH_3$), 3.04 and 3.27 (each 1H, dd, *J* 18 and 9 Hz, 4-$\underline{CH_2}$), 4.77 (1H, brt, *J* 9 Hz, 5-CH), 5.23 and 5.53 (each 1H, d, *J* 13.5 Hz, $CH_2CO_2$), 6.43 (1H, qd, *J* 7 and 1.5 Hz, $CH_3C\underline{H}$), 7.17 and 8.19 (each 2H, d, *J* 8.5 Hz, $C_6H_4$—$NO_2$).

## Example 5d

Sodium salt of p-nitrobenzyl (5R,6R)-3-ethylthio-6-[(S)-1-hydroxysulphonyloxyethyl]-7-oxo-1-aza-bicyclo[3.2.0]hept-2-ene-2-carboxylate

A solution of the diester (e16) (150 mg) in DMF (2 ml) was stirred at room temperature in the presence of sodium iodide (172 mg) for 4.5 h. The solution was evaporated to dryness and the residue chromatographed on silica gel using a gradient elution from chloroform to 25% ethanol in chloroform. Fractions containing the required product (t.l.c. and u.v.) were combined and evaporated in vacuo to afford the title compound as a solid (23 mg); $\lambda_{max.}$ (EtOH) 318 and 267 nm, ($H_2O$) 317 and 275 nm; $\nu_{max.}$ (KBr) 1765 and 1695 $cm^{-1}$.

## Example 6

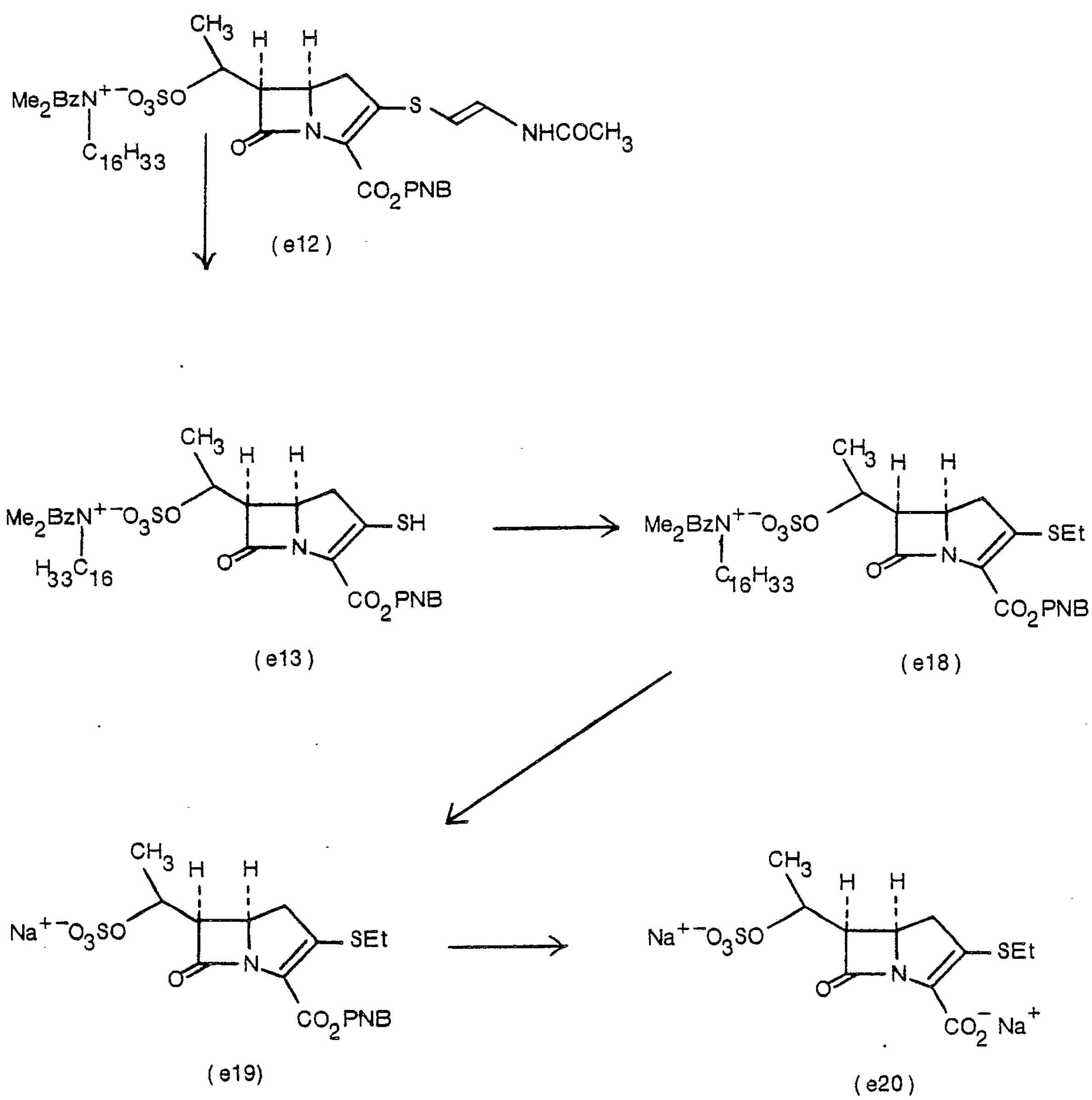

(e12)

(e13)

(e18)

(e19)

(e20)

Example 6a

Benzyldimethyl-n-hexadecylammonium salt of p-nitrobenzyl (5R,6R)-3-mercapto-6-[(S)-1-hydroxy-sulphonyloxyethyl]-7-oxo-1-azabicyclo[3.2.0]hept-2-ene-2-carboxylate

The quaternary ammonium salt (e12) (660 mg) was converted into the thiol (e13) by the method described in Example 4a.

Example 6b

Benzyldimethyl-n-hexadecylammonium salt of p-nitrobenzyl (5R,6R)-3-ethylthio-6-[(S)-1-hydroxy-sulphonyloxyethyl]-7-oxo-1-azabicyclo[3.2.0]hept-2-ene-2-carboxylate

The thiol derivative (e13) was treated with ethyl iodide and potassium carbonate in DMF in a manner analogous to that described in Example 4b. The title ethylthio-derivative (e18) was otbained as a foam (240 mg); $v_{max.}$ ($CHCl_3$) 1780 and 1700 $cm^{-1}$; $\lambda_{max.}$ (EtOH) 317 and 267 nm.

Example 6c

Sodium Salt of p-nitrobenzyl (5R,6R)-3-ethylthio-6-[(S)-1-hydroxysulphonyloxyethyl]-7-oxo-1-azabicyclo-[3.2.0]hept-2-ene-2-carboxylate

A column (10 × 2.5 cm) of Amberlyst A26 resin was washed successively with 100 ml quantities of methanol, water, ethanol and 30% $CHCl_3$ in EtOH. A solution of the quaternary ammonium salt (e18) (190 mg) in 30% $CHCl_3$ in EtOH (5 ml) was loaded on to the column which was then eluted successively with 100 ml portions of 30% $CHCl_3$ in EtOH, ethanol and water. Finally elution with a mixture (1:1) of 5% NaCl in $H_2O$ and methanol afforded a solution of the mono sodium salt (e19). This may be further purified by silica gel chromatography as described in Example 5d.

Example 6d

Disodium (5R,6R)-3-ethylthio-6-[(S)-1-sulphonato-oxyethyl]-7-oxo-1-azabicyclo[3.2.0]hept-2-ene-2-carboxylate

5% Pd on C catalyst (15 mg) was shaken with hydrogen in 30% aqueous dioxan (4 ml) for 30 min. A solution of the ester (e19) (7 mg) in 30% aqueous dioxan (1 ml) was introduced into the vessel and hydrogenation was continued for 3 h. $NaHCO_3$ (2 mg) was added to the mixture which was filtered over Celite, washing the pad with water (10 ml), and the solution was concentrated in vacuo to *ca.* 10 ml. The aqueous solution was washed with ethyl acetate (3 × 15 ml) and then concentrated in vacuo to *ca.* 3 ml. The solution was loaded onto a column of Biogel P2 (2.5 × 10 cm) which was then eluted with water. Fractions containing the title disodium salt (e20) were identified by the absorption in the UV spectrum at $\lambda_{max.}$ 298 nm.

*Demonstration*

Antibacterial effectiveness of the compound (e20) and MM 13902 in agar.

| Organism | | (e20) | MM 13902 |
|---|---|---|---|
| Escherichia coli | JT1 | ≤0.1 | ≤0.1 |
| | NCTC 15418 | ≤0.1 | ≤0.1 |
| | E8 | ≤0.1 | ≤0.1 |
| | 5 | ≤0.1 | ≤0.1 |
| | Ba78R+ | 0.2 | 0.8 |
| | JT4R+ | 0.4 | 1.6 |
| | JT20R+ | 0.8 | 1.6 |
| | E96R+ | ≤0.1 | 0.2 |
| | JT425C+ | ≤0.1 | 0.8 |
| | JT414C+ | 0.2 | 0.4 |
| Klebsiella aerogenes | T219 | ≤0.1 | ≤0.1 |
| | R112 | ≤0.1 | 0.2 |
| | I281 | ≤0.1 | 0.2 |
| | Va2R+ | 0.8 | 6.2 |
| Proteus mirabilis | T318 | 0.4 | ≤0.1 |
| | 889 | 0.8 | 0.2 |
| Proteus morganii | I | 0.4 | 0.2 |
| | T361 | 0.4 | 0.2 |
| Proteus rettgeri | I | 0.4 | 0.2 |
| | R110 | 0.2 | 0.2 |
| Proteus vulgaris | X | 0.4 | 0.4 |
| | NCTC 4395 | 0.2 | 0.1 |

| Organism | | (e20) | MM 13902 |
|---|---|---|---|
| Enterobacter cloacae | T749 | 6.2 | 12.5 |
| | NCTC 10005 | 6.2 | 12.5 |
| Enterobacter aerogenes | T660 | 0.2 | 0.4 |
| | NCTC 10006 | 0.4 | 0.4 |
| Citrobacter freundii | W18 | 1.6 | 6.2 |
| | T221 | 1.6 | 6.2 |
| Serratia marcescens | SM27 | 0.4 | 1.6 |
| | Wo146 | 1.6 | 3.1 |
| Pseudomonas aeruginosa | W975 | 100 | 50 |
| | NCTC 10662 | 50 | 25 |
| | Dalgleish | 50 | 50 |
| Staphylococcus aureus | Smith | 0.4 | 1.6 |
| | ATCC 25923 | 0.4 | 1.6 |
| | ME9+ | 1.6 | 3.1 |
| | T67+ | 0.8 | 0.8 |
| | T150+ | 0.4 | 1.6 |
| Streptococcus faecalis | C90 | 12.5 | 6.2 |
| | T1101 | 12.5 | 6.2 |
| Bacteroides fragilis | 2118 | 0.2 | 0.2 |
| | B3 | 0.4 | 0.4 |
| | WS12 | 0.2 | 0.2 |
| | BC16 | 1.6 | 1.6 |
| | VP1 8249 | 0.4 | 0.2 |
| | WS41 | 0.2 | 0.4 |
| | WS1 | 6.2 | 6.2 |
| | BC4 | 0.8 | 1.6 |

Example 7

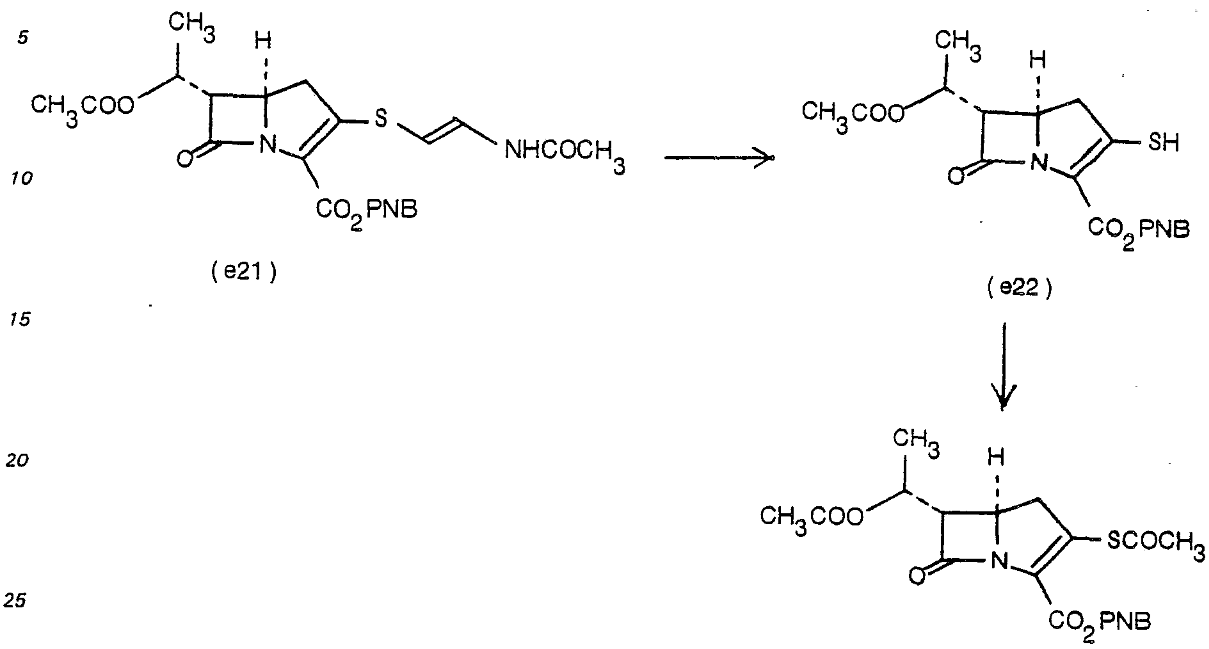

(e21)

(e22)

(e23)

Example 7a

p-Nitrobenzyl (5R,6S)-3-mercapto-6-[(S)-1-acetoxyethyl]-7-oxo-1-azabicyclo[3.2.0]hept-2-ene-2-carboxylate

The acetoxy derivative (e21) (90 mg) was dissolved in acetone (2 ml) containing water (3 drops). The solution was cooled to −20° and a solution of N-bromoacetamide in acetone (0.5 ml) was added with stirring. After 25 min at −20° the solution was diluted with chloroform (25 ml) and washed with water (25 ml). The organic layer was dried ($MgSO_4$) and concentrated in vacuo to afford a gum, which contained the thiol (e22), $\nu_{max.}$ ($CHCl_3$) 1780, 1730 and 1700 sh $cm^{-1}$.

Example 7b

p-Nitrobenzyl (5R,6S)-3-acetylthio-6-[(S)-1-acetoxyethyl]-7-oxo-1-azabicyclo[3.2.0]hept-2-ene-2-carboxylate

The product (e22) was dissolved in pyridine (1 ml) and to the stirred solution was added acetyl chloride (3 drops). After 10 min the mixture was evaporated to dryness and the product was partitioned between chloroform (25 ml) and water (25 ml). The organic layer was washed with pH 3 phosphate buffer (20 ml), dilute aqueous sodium bicarbonate (20 ml) and water (20 ml), and then dried ($MgSO_4$) and concentrated in vacuo. The residue was chromatographed on silica gel using 50% petroleum ether-ethyl acetate to elute. The first-eluted component was the title diacetyl derivative (e23) (5 mg); $\nu_{max.}$ ($CHCl_3$) 1790, 1740 and *ca.* 1700 sh $cm^{-1}$; $\nu_{max.}$ (EtOH) *ca.* 300 sh and 267 nm. δ($CDCl_3$) *inter alia* 1.43 (3H, d, *J* 6.5 Hz, $C\underline{H}_3CH$), 2.11 (3H, s, $CH_3CO$), 2.40 (3H, s, $COCH_3$) and *ca.* 5.15 (1H, m, $CH_3C\underline{H}$).

Example 8

(e12)→(e13)→(e18)→(e19)→(e20)

Example 8a

Benzyldimethyl-n-hexadecylammonium salt of p-nitrobenzyl (5R,6R)-3-mercapto-6-[(S)-1-hydroxysulphonyloxyethyl]-7-oxo-1-azabicyclo[3.2.0]hept-2-ene-2-carboxylate

The derivative (e12) (3.5 g) was converted into the thiol (e13) by the method of Example 6, step A, of EP—B—0 024 832.

Example 8b

Benzyldimethyl-n-hexadecylammonium salt of p-nitrobenzyl (5R,6R)-3-methylthio-6-[(S)-1-hydroxysulphonyloxyethyl]-7-oxo-1-azabicyclo[3.2.0]hept-2-ene-2-carboxylate

The thiol (e13) was treated with ethyl iodide in the manner as described in Example 4b to afford the ethylthio-derivative (e18) (1.48 g).

Example 8c

Sodium salt of p-nitrobenzyl (5R,6R)-3-ethylthio-6-[(S)-1-hydroxysulphonyloxyethyl]-7-oxo-1-azabicyclo-[3.2.0]hept-2-ene-2-carboxylate

A solution of the quaternary ammonium compound (e18) in acetone (15 ml) was mixed with a solution of sodium iodide (268 mg) in acetone (5 ml). A white solid crystallised out of the solution and this was filtered and washed with acetone and ether. The product (500 mg) consisted of the title compound (e19); $\lambda_{max.}$ ($H_2O$) 317 (12,470) and 273 nm (10,750); $\nu_{max.}$ (KBr) 1770 and 1695 $cm^{-1}$; δ(DMF-$d_7$) 1.27 (3H, t, *J* 7.5 Hz, $CH_3CH_2$), 1.46 (3H, d, *J* 6 Hz, $CH_3CH$), 2.94 (2H, q, *J* 7.5 Hz, $CH_2CH_3$), 3.17 (1H, dd, *J* 19 and 9.5 Hz, 4-$CH_a$), 3.71 (1H, dd, *J* 5.5 and 11 Hz, 6-CH), 3.95 (1H, dd, *J* 19 and 8.5 Hz, 4-$CH_b$), *ca.* 4.3 (1H, m, 5-CH), *ca.* 4.55 (1H, m, $CH_3CH$), 5.31 and 5.55 (each 1H, d, *J* 13.5, $CH_2Ar$) and 7.80 and 8.27 (each 2H, d, *J* 8.5 Hz, $C_6H_4$-$NO_2$).

Example 8d

Disodium salt of (5R,6R)-3-ethylthio-6-[(S)-1-hydroxysulphonyl-oxyethyl]-7-oxo-1-azabicyclo[3.2.0]hept-2-ene-2-carboxylate

The ester (e19) (1.12 g) was hydrogenolysed with 5% Pd-C (1.5 g) in 30% aqueous dioxan (100 ml) using the procedure of Example 6d. After work-up (190 mg $NaHCO_3$), as therein described, and chromatography on a column of Diaion HP20 (3.5 × 15 cm), eluting with water, fractions containing the product were combined and freeze-dried. The resulting solid (460 mg) consisted of the title disodium salt (e20); $\lambda_{max.}$ ($H_2O$) 300 nm (7900); $\nu_{max.}$ (KBr) 1750 and 1595 $cm^{-1}$; $[\alpha]_D$ (c.1, $H_2O$) +32°; δ ($D_2O$) 1.20 (3H, t, *J* 7 Hz, $CH_3CH_2$), 1.45 (3H, d, *J* 6.5 Hz, $CH_3CH$), 2.80 (2H, q, *J* 7 Hz, $CH_2CH_3$), 3.04 (1H, dd, *J* 10 and 18 Hz, 4-$CH_a$), 3.35 (1H, dd, *J* 9 and 18 Hz, 4-$CH_b$), 3.78 (1H, dd, *J* 9 and 5 Hz, 6-CH), *ca.* 4.2 (1H, m, 5-CH), and *ca.* 4.75 (1H, m, $CH_3CH$).

Example 9

p-Nitrobenzyl (5R,6R)-3-(2-p-nitrobenzyloxycarbonylaminoethylthio)-6-[(S)-1-ethylthioethyl]-7-oxo-1-azabicyclo[3.2.0]hept-2-ene-2-carboxylate

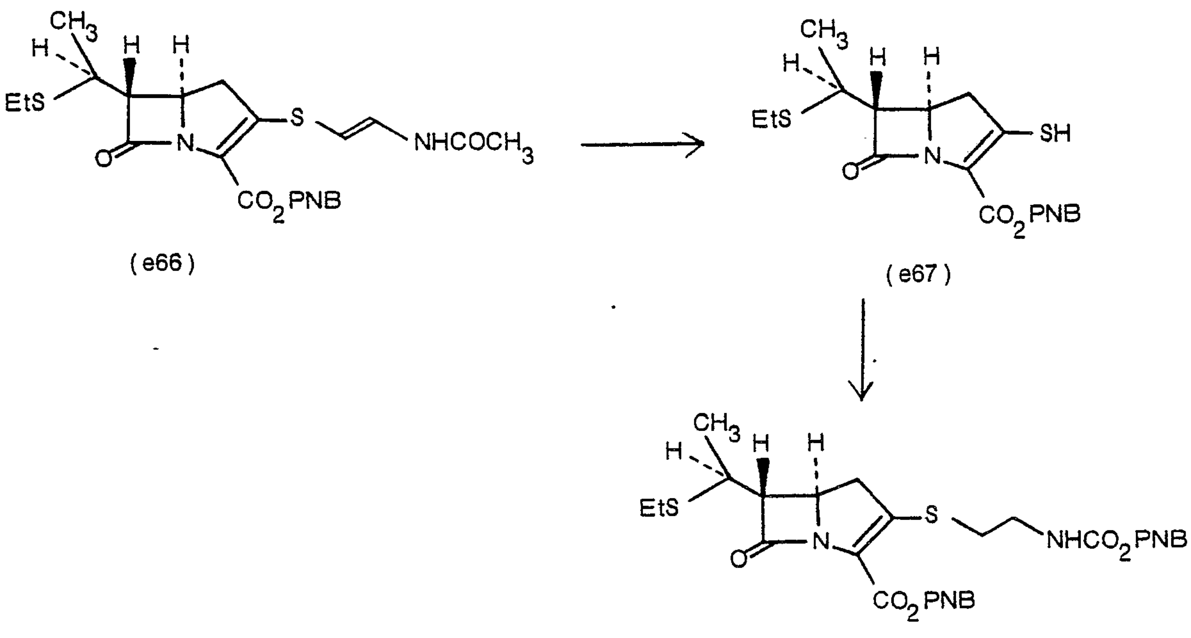

(e66) (e67) (e68)

The ethylthio-derivative (e66) (270 mg) was treated with N-bromoacetamide (77 mg) as described in Example 2, step A, of EP—B—0 024 832 to afford the thiol (e67). Alkylation of (e67) with 2-p-nitrobenzyloxy-carbonylaminoethyl bromide (338 mg) and potassium carbonate in DMF, in a similar manner to that described in Example 1b, afforded the title derivative (e68) (27 mg); $\lambda_{max.}$ (EtOH) 319 nm.; $\nu_{max.}$ ($CH_2Cl_2$) 3450, 1780 and 1705 sh $cm^{-1}$ δ ($CDCl_3$) 1.27 (3H, t, *J* 7 Hz, $CH_3CH_2$), 1.39 (3H, d, *J* 6.5 Hz, $CH_3CH$), 2.61 (2H, q, *J* 7 Hz, $CH_2S$), 2.90—3.60 (8H, m, 4-$CH_2$, $SCH_2CH_2N$, 6-CH and $CHCH_3$), 4.16 (1H, m, 5-CH), 5.20 (2H, s, $CH_2Ar$), *ca.* 5.25 (1H, br, NH), 5.23 and 5.52 (each 1H, d, *J* 14 Hz, $ArCH_2$), 7.48 and 7.64 (each 2H, d, J 14 Hz, aromatic protons) and 8.20 (4H, d, *J* 14 Hz, aromatic protons).

Example 10

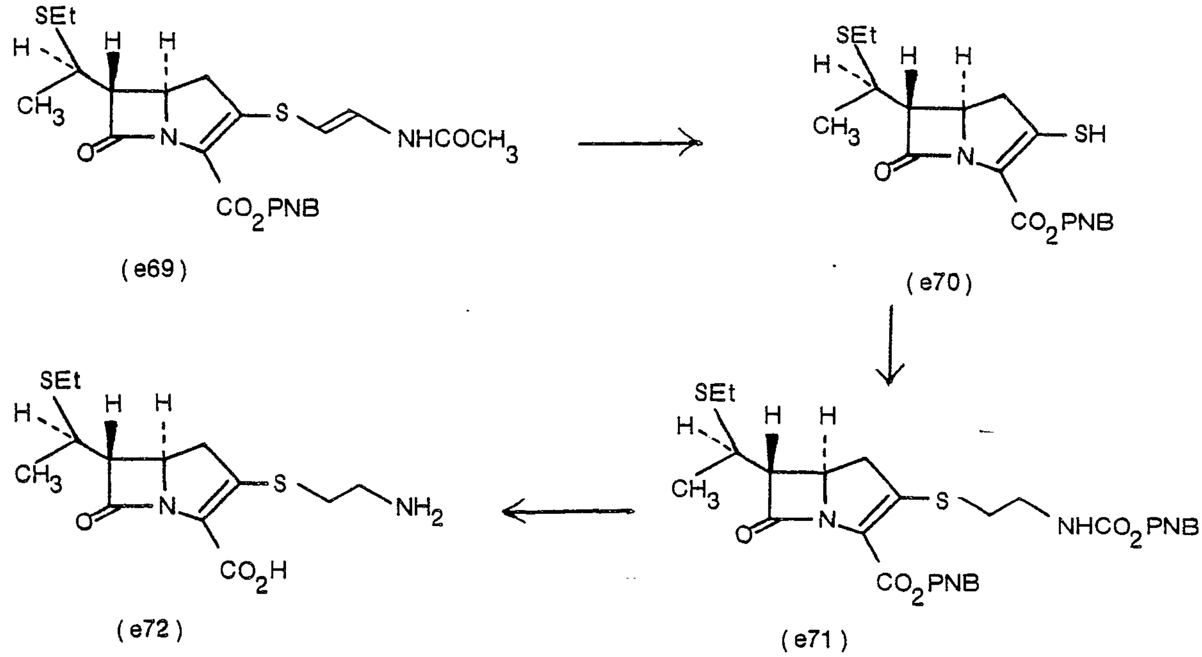

(e69) (e70) (e71) (e72)

Example 10a

p-Nitrobenzyl (5R,6R)-3-(2-p-nitrobenzyloxycarbonylaminoethylthio)-6-[(S)-1-ethylthioethyl]-7-oxo-1-azabicyclo[3.2.0]hept-2-ene-2-carboxylate

Compound (e69) (600 mg) was converted, via the thiol (e70), into the derivative (e71) by use of the methods outlined in Example 9. The title compound (e71) was obtained as a crystalline solid (131 mg); m.p. 205—206°: $\lambda_{max.}$ (EtOH) 319 (13,125) and 266 (21,235); $\nu_{max.}$ (KBr) 1775 and 1725—1700 br $cm^{-1}$; δ (DMF-$d^7$) 1.22 (3H, t, *J* 7 Hz, $C\underline{H}_3CH_2$), 1.41 (3H, d, *J* 6.5 Hz, $C\underline{H}_3CH$), 2.64 (2H, q, *J* 17 Hz, $CH_2S$), *ca.* 3.0—3.6 (8H, m, 4-$CH_2$, 6-CH, $CH_3C\underline{H}$ and $SCH_2CH_2N$), 4.17 (1H, m, 5-CH), 5.25 (2H, s, $C\underline{H}_2Ar$), 5.33 and 5.58 (each 1H, d, $C\underline{H}_2Ar$), *ca.* 7.65 (1H, br, NH), 7.67 and 7.82 (each 2H, d, *J* 9 Hz, aromatic protons) and 8.27 (4H, d, *J* 9 Hz, aromatic protons). [Found: c, 53.4; H, 4.8; N, 8.8%. $C_{28}H_{30}N_4O_9S_2$ requires C, 53.3; H, 4.8; N, 8.9%].

Example 10b

(5R,6R)-3-(2-Aminoethylthio)-6-[(R)-1-ethylthioethyl]-7-oxo-1-azabicyclo[3.2.0]hept-2-ene-2-carboxylic acid

A mixture of the thioether (e71) (100 mg), 5% Pd on C (150 mg), 0.05 M, pH 7 phosphate buffer (5 ml) and dioxan (12 ml) was shaken in an atmosphere of hydrogen for 2 h. A further quantity (100 mg) of 5% Pd on C was added and hydrogenation was continued for a further 2.25 h. The mixture was filtered through Celite, washing the pad well with water (30 ml); and the filtrate was evaporated in vacuo to a volume of *ca.* 20 ml. The aqueous solution was washed with ethyl acetate (3 × 30 ml) and then further concentrated in vacuo to a volume of *ca.* 15 ml. This solution contained the title zwitterionic derivative (e72); $\lambda_{max.}$ ($H_2O$) 298 nm.

Example 11

p-Nitrobenzyl (5R,6R)-3-methylthio-6-(1-ethylthioethyl)-7-oxo-1-azabicyclo[3.2.0]hept-2-ene-2-carboxylate

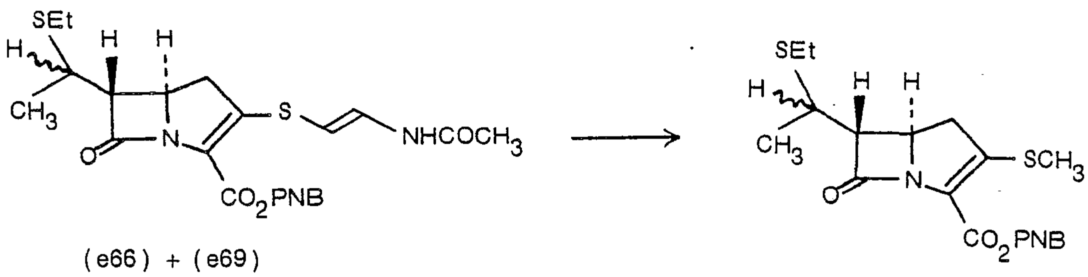

(e66) + (e69) (e73)

A mixture of the esters (e66) and (e69) (200 mg) was converted into derivative (e73) using the methodology of Example 2 of EP—B—0 024 832, with iodomethane as the alkylating agent in part b. The

title compound (e73) was obtained as a gum (50 mg); $\nu_{max.}$ ($CH_2Cl_2$) 1780 and 1705 $cm^{-1}$; δ ($CDCl_3$) *ca.* 1.15—1.50 (3H, m, $C\underline{H}_3CH_2$), 2.38 (3H, s, $CH_3S$), 2.59 (2H, q, *J* 7 Hz, $SC\underline{H}_2CH_3$), *ca.* 2.85—03.60 (4H, m, 4-$CH_2$, 6-CH and $CH_3C\underline{H}$), 4.15 (1H, m, 5-CH), 5.22 and 5.52 (each 1H, d, *J* 14 Hz, $C\underline{H}_2Ar$), 7.66 and 8.22 (each 2H, d, *J* 9 Hz, $\underline{Ar}CH_2$) [$\underline{M}$+, 422].

Example 12

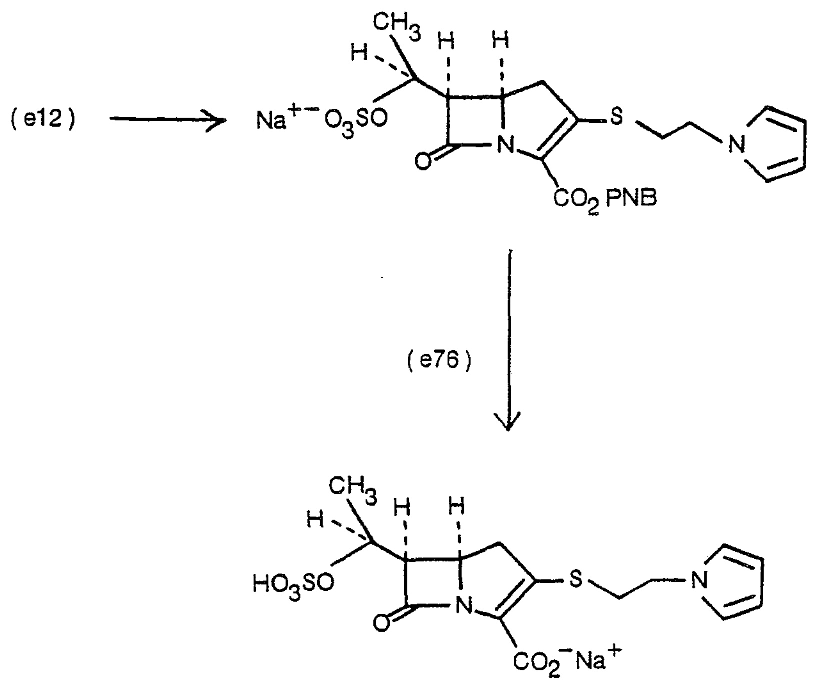
(e12)
(e76)

(e77)

Example 12a and 12b

The method of Example 8 was used to convert the quaternaryammonium salt (e12) (2.8 g) into the thiol (e13), which was then alkylated with 1-(2-bromethyl) pyrrole.

Example 12c

Sodium salt of p-nitrobenzyl (5R,6R)-3-(2-pyrrol-1-ylethyl)-6-[(S)-1-hydroxysulphonyloxyethyl]-7-oxo-1-azabicyclo[3.2.0]hept-2-ene-2-carboxylate

To a solution of the product from example 12b in acetone (10 ml) a solution of sodium iodide (124 mg) in acetone (5 ml) was added. The resulting solution was cooled and the solid formed (quaternaryammonium iodide) was removed by filtration. The filtrate was concentrated in vacuo and then chromatographed on silica using a gradient elution of $CHCl_3$ to 30% EtOH-$CHCl_3$. The requisite fractions were combined and evaporated in vacuo to afford the title compound (e76) (196 mg); $\lambda_{max.}$ ($H_2O$) 316 and 273 nm; $\nu_{max.}$ (KBr) 1765 and 1700 $cm^{-1}$; δ (DMF-$d_7$) 1.48 (3H, d, *J* 6.5 Hz, $CH_3CH$), *ca.* 3.2 (1H, m, 4-$CH_a$), 3.28 (2H, m, $SCH_2$), *ca.* 3.7 (2H, m, 6-CH and 4-$CH_b$), 4.22 (2H, m, $CH_2N$), *ca.* 4.55 (2H, m, 5-CH and $C\underline{H}CH_3$), 5.31 and 5.55 (each 1H, d, *J* 14 Hz, $C\underline{H}_2Ar$) 6.00 (2H, m, pyrrole ß-CH), 6.91 (2H, m, pyrrole α-CH), 7.80 and 8.27 (each 2H, d, *J* 9 Hz $\underline{Ar}CH_2$).

Example 12d

Monosodium salt of (5R,6R)-3-(2-Pyrrol-1-ylethylthio)-6-[(S)-1-hydroxysulphonyloxyethyl]-7-oxo-1-azabicyclo[3.2.0]hept-2-ene-2-carboxylic acid

The procedure of Example 5 of EP—B—0 024 832 was used to hydrogenolyse the ester (e76) (150 mg). Chromatography of Biogel P2 (10 × 2.5 cm) afforded the title compound (e77) (46 mg). The solution was freeze-dried to give a solid; $\lambda_{max.}$ ($H_2O$) 300 nm.; $\nu_{max.}$ (KBr) 1750 and 1600 br $cm^{-1}$.

Example 13

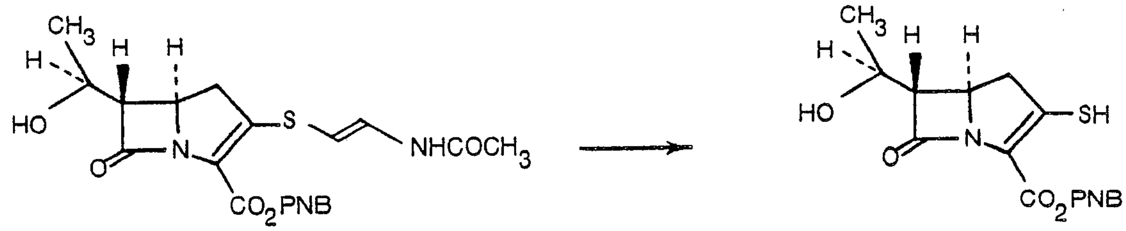

(e1) (e2)

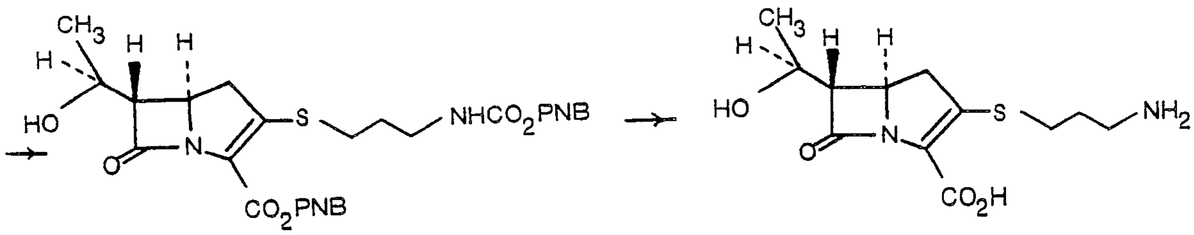

(e78) (e79)

Example 13a

p-Nitrobenzyl (5R,6S)-3-mercapto-6[(S)-1-hydroxyethyl]-7-oxo-1-azabicyclo[3.2.0]hept-2-ene-2-carboxylate

The ester (e1, 500 mg, 1.119 mM) was dissolved in 1,4-dioxan (10 ml) containing water (30 drops). A solution of N-bromoacetamide (155 mg, 1.119 mM) in 1,4-dioxan was then added and the solution stirred at room temperature for 4.5 minutes. Chloroform (50 ml) was added and the organic phase was washed with pH 7.0, 0.05 M phosphate buffer, saturated sodium chloride solution and dried over anhydrous magnesium sulphate. Filtration and removal of the solvent at reduced pressure yielded the crude thiol (e2) as a gum, $\nu_{max.}$ ($CHCl_3$) 1780, 1730 and 1700 $cm^{-1}$.

Example 13b

p-Nitrobenzyl (5R,6S)-3-[3-(p-nitrobenzyloxycarbonylamino)propylthio]-6-[(S)-1-hydroxyethyl]-7-oxo-1-azabicyclo[3.2.0]hept-2-ene-2-carboxylate

The crude thiol was dissolved in dry dimethylformamide (10 ml) and stirred at room temperature for 25 minutes with anhydrous potassium carbonate (155 mg, 1.119 mM) and 3-(N-p-nitrobenzyloxycarbonyl)-amino-1-bromopropane (355 mg; 1.119 mM). Ethyl acetate was added and the organic solution washed with water, saturated sodium chloride solution and dried over anhydrous magnesium sulphate. After filtration the solvent was removed at reduced pressure to give a pale yellow oil, which was chromatographed over silica gel (50 gm). Elution with 5% ethanol/chloroform afforded the title compound (e78) (155 mg) as a white solid. This solid was digested in diethyl ether and collected by filtration (145 mg), m.p. 164—168°C, $\nu_{max.}$ (KBr) 3420, 1765, 1700, 1608 $cm^{-1}$; $\lambda_{max.}$ (EtOH) 320 nm (εm 13,400) and 266 nm (εm 20,050); δH ($d_7$-DMF) 1.28 (3H, d, $J$ 6.6 Hz, $C\underline{H}_3CH$), 1.86 (2H, m, $CH_2C\underline{H}_2CH_2$), 2.99 (2H, t, $SC\underline{H}_2$), 3.24 (t, $C\underline{H}_2NH$), 3.33 (d, 4-$C\underline{H}_2$), 3.49 (1H, t, $J_{6-8}$ 4.3 Hz, 6-C$\underline{H}$), 4.05 (1H, m, $CH_3C\underline{H}$), 4.24 (1H, dt, $J_{4-5}$ 9.0 Hz, $J_{5-6}$ 2.9 Hz, 5-C$\underline{H}$), 5.12 (1H, d, $J_{OH-8}$ 4.6 Hz, O$\underline{H}$), 5.23—5.65 (4H, S+AB, 2 × $C\underline{H}_2Ar$), 7.39 (1H, broad resonance, N$\underline{H}$), 7.68 (2H, d, Ar protons), 7.82 (2H, d, Ar protons), 8.25 (4H, d, Ar protons); m/e 286, 153, 136, no $M^+$; (Found: C, 53.71; H, 4.60; N, 9.07. $C_{27}H_{28}N_4O_{10}S$ requires: C, 53.99; H, 4.70; N, 9.33%).

Example 13c

(5R,6S)-3-(3-Aminopropylthio)-6-[(S)-1-hydroxyethyl]-7-oxo-1-azabicyclo[3.2.0]hept-2-ene-2-carboxylic acid

A solution of the ester (e78), (130 mg) in 1,4-dioxan (15 ml), water (4.5 ml), ethanol (1.35 ml) and 0.05 M pH 7.0 phosphate buffer (6 ml) was hydrogenated in the presence of 10% palladium on carbon (200 mg) for 2 hours. The suspension was then filtered over Celite, washing with water (20 ml). The filtrate was concentrated to about 20 ml and washed with ethyl acetate (3 × 25 ml). The aqueous solution was further concentrated to about 10 ml and chromatographed on a column of XAD-2, eluting with water. Fractions containing the product were combined to yield the title compound (e79) (14.5 mg) in aqueous solution, $\lambda_{max.}$ ($H_2O$) 299 nm.

Example 14

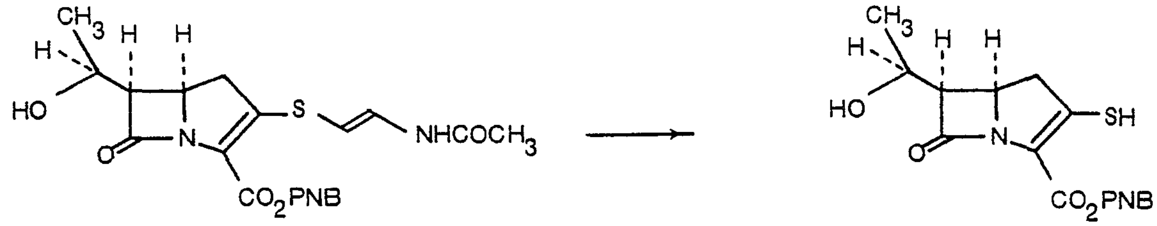

(e28) (e40)

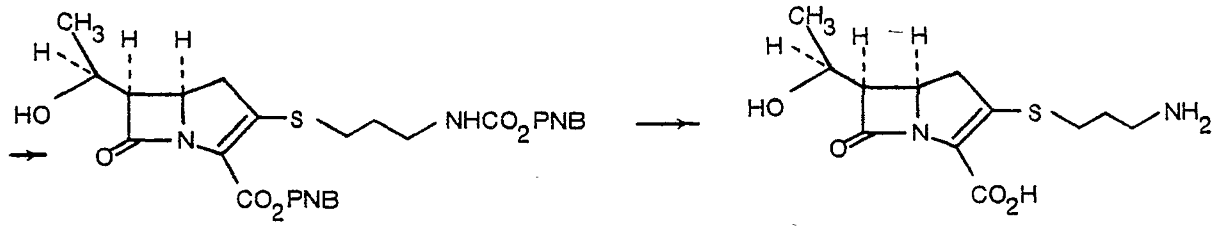

(e80) (e81)

Example 14a

p-Nitrobenzyl (5R,6R)-3-[3-(p-nitrobenzyloxycarbonylamino)propylthio]-6-[(S)-1-hydroxyethyl]-7-oxo-1-azabicyclo[3.2.0]hept-2-ene-2-carboxylate

The title compound (e80) was prepared as a white solid in 20% yield from the ester (e28), adopting the procedure described in Example 13., m.p. 104—106°C (ethyl acetate/diethyl ether), $\lambda_{max.}$ (EtOH) 317 nm (Em 12,360) 266 nm (Em 18,890); $\nu_{max.}$ (KBr) 3410, 1770, 1700, 1608 $cm^{-1}$; δH ($d_7$-DMF) 1.31 (3H, d, $J$ 6 Hz, $C\underline{H}_3CH$), 1.9 (2H, m, $CH_2C\underline{H}_2CH_2$), 3.03 (2H, t, $SC\underline{H}_2$), 3.22 (t, $C\underline{H}_2NH$), 3.35 (d, 4-$C\underline{H}_2$), 3.6 (m, 1H, 6-$C\underline{H}$), 3.9—4.5 (2H, m, 8-$C\underline{H}$ + 5-$C\underline{H}$), 5.06 (1H, d, $J$ 6 Hz, $O\underline{H}$), 5.23 (2H, S, $C\underline{H}_2Ar$), 5.44 (2H, AB, $C\underline{H}_2Ar$), 7.4 (1H, broad resonance, $N\underline{H}$), 7.6—8.4 (8H, 2 × AB, 2 × $\underline{A}r$); m/e 555, 514, 286, 136, no $M^+$.

Example 14b

(5R,6R)-3-(3-Aminopropylthio)-6-[(S)-1-hydroxyethyl]-7-oxo-1-azabicyclo[3.2.0]hept-2-ene-2-carboxylic acid

The ester (e80) was hydrogenolysed as in Example 13c to yield (5R,6R)-3-(3-aminopropylthio)-6-[(S)-1-hydroxyethyl]-7-oxo-1-azabicyclo[3.2.0]hept-2-ene-2-carboxylic acid (e81) in 26% yield as an aqueous solution (based on Em 8,500). A sample was freeze dried to give a pale yellow fluffy solid, $\lambda_{max.}$ ($H_2O$) 292 nm, $\nu_{max.}$ (KBr) 3420, 1750, 1600 (broad) $cm^{-1}$.

Example 15

(e1) 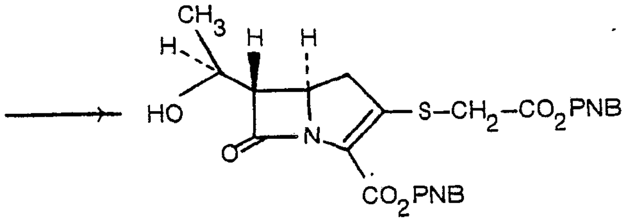

(e82)

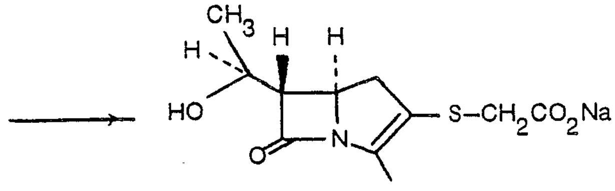

(e83)

### Example 15a

p-Nitrobenzyl (5R,6S)-3-(p-nitrobenzyloxycarbonylmethylthio)-6-[(S)-1-hydroxyethyl]-7-oxo-1-azabicyclo-[3.2.0]hept-2-ene-2-carboxylate

The diester (e82) (150 mg) was prepared as a pale yellow solid by reaction of the ester (e1) (500 mg) with N-bromoacetamide, followed by p-nitrobenzyl bromoacetate, utilising the procedure outlined in Example 13., $\lambda_{max.}$ (EtOH) 309 nm, 265 nm (Em 15,355); $\nu_{max.}$ (KBr) 3500, 1765, 1737, 1700, 1608, 1550, 1520, 1348 $cm^{-1}$; δH ($d_7$-DMF) 8.26 (d) + 7.79 (t, 8H, aromatic protons), 5.40 (s + q, 4H, C<u>H</u>$_2$Ar protons); 5.20 (1H, d, O<u>H</u>), 4.0—4.5 (s + m, 4H, C<u>H</u>$_2$$CO_2$PNB + 5-C<u>H</u> + 8-C<u>H</u>), 1.27 (3H, d, C<u>H</u>$_3$CH); m/e 513, 286, 168, 136, 78 no $M^+$.

### Example 15b

Disodium (5R,6S)-3-(carboxymethylthio)-6-[(S)-1-hydroxyethyl]-7-oxo-1-azabicyclo[3.2.0]hept-2-ene-2-carboxylate

5% Palladium on carbon catalyst (75 mg) was shaken with hydrogen in 30% aqueous 1,4-dioxan (10 ml) at ambient pressure and temperature for 0.5 hours. A solution of the diester (e82) (50 mg) in 1,4-dioxan (10 ml) was added and the hydrogenation was continued for a further 3.5 hours. Sodium bicarbonate (15 mg) was added and the mixture was filtered through Celite, washing well with water (30 ml). The filtrate was concentrated at reduced pressure to approximately 20 ml and washed with ethyl acetate (3 × 25 ml). The aqueous solution was further concentrated to about 10 ml and chromatographed on a column of Biogel P-2, eluting with water. Fractions containing the title compound (e83) were identified by the chromophore at $\lambda_{max.}$ ($H_2O$) 300 nm in the U.V. spectrum. These fractions were combined and freeze dried to afford a pale yellow solid (9 mg).

### Example 16

(e28)

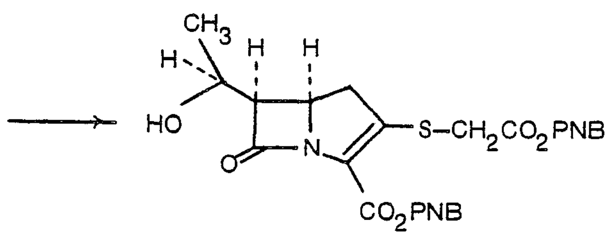

(e84)

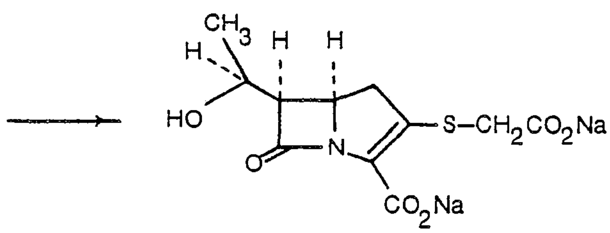

(e85)

### Example 16a

p-Nitrobenzyl (5R,6R)-3-(p-nitrobenzyloxycarbonylmethylthio)-6-[(S)-1-hydroxyethyl]-7-oxo-1-azabicyclo-[3.2.0]hept-2-ene-2-carboxylate

The diester (e84) (179 mg) was prepared as a pale yellow solid by reaction of the thiol (e40), derived from the ester (e28) (500 mg) with p-nitrobenzyl bromoacetate, by the method described in Example 13, $\lambda_{max.}$ (EtOH) 311 nm (Em 11,120), 265 nm (Em 21,197); $\nu_{max.}$ (KBr) 3490, 1760 (broad), 1700, 1608, 1555, 1520, 1348 $cm^{-1}$., $\delta_H$ ($d_7$-DMF) 8.26 (d) and 7.78 (t, 8H, aromatic protons), 5.40 (4H, q + s, C<u>H</u>$_2$Ar protons), 5.03 (1H, d, O<u>H</u>), 3.8—4.5 (s + m, 4H, C<u>H</u>$_2$$CO_2$PNB + 5-C<u>H</u> + 8-C<u>H</u>), 3.2—3.8 (m, 4-C<u>H</u>$_2$ + 6-C<u>H</u>), 1.28 (3H, d, C<u>H</u>$_3$CH); m/e 513, 471, 286, 227, 168, 136, no $M^+$.

### Example 16b

Disodium (5R,6R)-3-(carboxymethylthio)-6-[(S)-1-hydroxyethyl]-7-oxo-1-azabicyclo[3.2.0]hept-2-ene-2-carboxylate

The diester (e84) (75 mg) was hydrogenolysed in the manner described in Example 15b. Biogel P-2 column chromatography, eluting with water afforded the title compound (e85) (15 mg) in aqueous solution, $\lambda_{max.}$ ($H_2O$) 300 nm. A sample was freeze dried to yield a yellow solid.

Example 17

(e1)
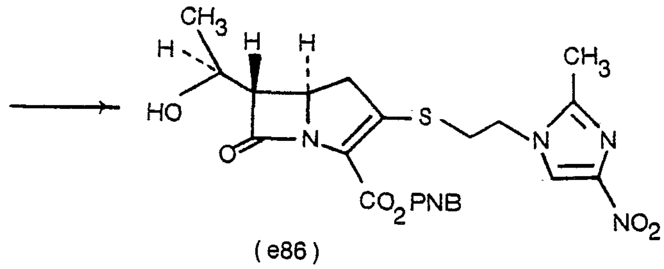
(e86)

(e1)
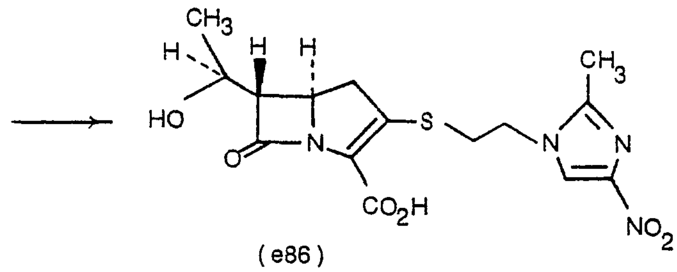
(e86)

Example 17a

p-Nitrobenzyl (5R,6S)-3-[2-(2-methyl-4-nitroimidazol-1-yl)ethylthio]-6-[(S)-1-hydroxyethyl]-7-oxo-1-azabicyclo[3.2.0]hept-2-ene-2-carboxylate

The title compound (e86) (97 mg) was prepared as a pale yellow solid by reacting the thiol (e2), derived from ester (e1) (500 mg) with 1-(ß-bromoethyl)-2-methyl-4-nitroimidazole, adopting the procedure described in Example 13, $\lambda_{max.}$ (EtOH) 308 m, (Em 15449), 270 nm (Em 13539), $\nu_{max.}$ (KBr) 3420, 1770, 1700, 1605, 1540, 1520, 1345, 1333, 750, 738 $cm^{-1}$, $\delta_H$ ($d_7$-DMF) 8.39 (1H, S, imidazole-C<u>H</u>), 8.25 (2H, d, aromatic protons), 7.81 (2H, d, aromatic protons), 5.44 (2H, q, C<u>H</u>$_2$Ar), 5.14 (1H, d, O<u>H</u>), 4.43 (2H, t, C<u>H</u>$_2$N), (dt, 5-CH), 2.43 (3H, s, imidazole-C<u>H</u>$_3$), 1.27 (3H, d, C<u>H</u>$_3$CH), m/e 330.0846 ($C_{16}H_{14}N_2O_6$ requires 330.0852), 286, 187, 128, 44, no $M^+$.

Example 17b

(5R,6S)-3-[2-(2-Methyl-4-amino-imidazol-1-yl)ethylthio]-6-(S)-1-hydroxysulphonyloxyethyl]-7-oxo-1-azabicyclo[3.2.0]hept-2-ene-2-carboxylic acid

Hydrogenolysis of the ester (e86) (70 mg) by the procdure described in Example 13c gave an aqueous solution containing (5R,6S)-3-[2-(2-methyl-4-amino-imidazol-1-yl)ethylthio]-6-[(S)-1-hydroxyethyl]-7-oxo-1-azabicyclo[3.2.0]hept-2-ene-2-carboxylic acid (87). A sample was freeze-dried to yield a white fluffy solid, $\lambda_{max.}$ ($H_2O$) 298 nm, $\nu_{max.}$ (KBr) 3400, 1750, 1665, 1590, 1400 $cm^{-1}$.

Example 18

(e28)
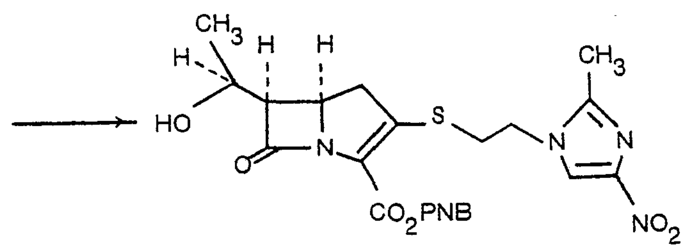
(e88)

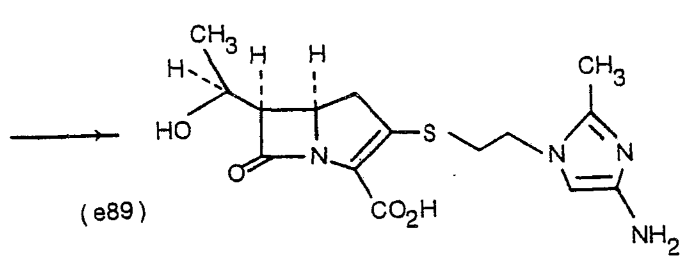
(e89)

Example 18a

p-Nitrobenzyl (5R,6R)-3-[2-(2-methyl-4-nitroimidazol-1-yl)ethylthio]-6-[(S)-1-hydroxyethyl]-7-oxo-1-azabicyclo[3.2.0]hept-2-ene-2-carboxylate

The title compound (e88) (85 mg) was prepared as a white solid by reacting the thiol (e40), derived from the ester (e28) (500 mg) with 1-(β-bromoethyl)-2-methyl-4-nitroimidazole, utilising the procedure described in Example 13. $\lambda_{max.}$ (EtOH) 308 nm (Em 11,961), 275 nm, $\nu_{max.}$ ($CHBr_3$) 3400, 1775, 1698, 1605, 1563, 1540, 1515, 1320, 743 $cm^{-1}$., $\delta_H$ ($d_7$-DMF) 8.39 (1H, S, imidazole C<u>H</u>), 8.27 (2H, d, aromatic protons), 7.81 (2H, S, aromatic protons), 5.45 (2H, q, $C\underline{H}_2$Ar protons), 4.45 (2H, t, $C\underline{H}_2N$), 2.44 (3H, S, imidazole $C\underline{H}_3$), 1.30 (3H, d, $C\underline{H}_3CH$).

Example 18b

(5R,6R)-3-[2-(2-Methyl-4-amino-imidazol-1-yl)ethylthio]-6-[(S)-1-hydroxyethyl]-7-oxo-1-azabicyclo[3.2.0]-hept-2-ene-2-carboxylic acid

Hydrogenolysis of the ester (e88) (60 mg) by the procedure described in Example 13c gave an aqueous solution containing the title compound (e89), $\lambda_{max.}$ ($H_2O$) 297 nm, $\nu_{max.}$ (KBr) 3400, 1750, 1665, 1595, 1400 $cm^{-1}$.

Example 19

(e1) 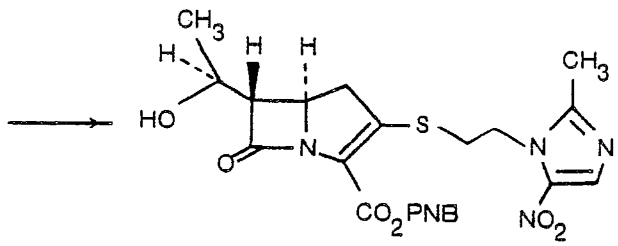

(e90)

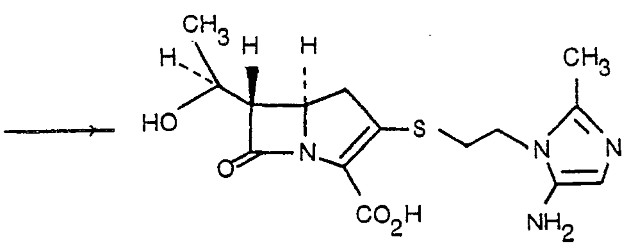

(e91)

Example 19a

p-Nitrobenzyl (5R,6S)-3-[2-(2-methyl-5-nitroimidazol-1-yl)ethylthio]-6-[(S)-1-hydroxyethyl]-7-oxo-1-azabicyclo[3.2.0]hept-2-ene-2-carboxylate

p-Nitrobenzyl (5R,6S) - 3 - [2 - methyl - 5 - nitroimidazol - 1 - yl)ethylthio] - 6 - [(S) - 1 - hydroxyethyl] - 7 - oxo - 1 - azabicyclo[3.2.0]hept - 2 - ene - 2 - carboxylate (e90) (40 mg) was obtained as a white solid by the reaction of the thiol (e2) derived from the ester (e1) (250 mg) with 1-(β-bromoethyl)-2-methyl-5-nitroimidazole, utilising the procedure described in Example 13., $\lambda_{max.}$ (EtOH) 312 nm (Em 17925), 268 nm (Em 12885), $\nu_{max.}$ (KBr) 3420, 1775, 1700, 1608, 1520, 1332, 1350, 1365, 742 $cm^{-1}$., $\delta_H$ ($d_7$-DMF) 8.25 (2H, d, aromatic protons), 8.01 (1H, s, imidazole C<u>H</u>), 7.80 (2H, d, aromatic protons), 5.44 (2H, q, $C\underline{H}_2$Ar), 5.19 (1H, d, O<u>H</u>), 4.68 (2H, t, $C\underline{H}_2N$), 4.2 (2H, dt + m, 5-C<u>H</u> + 8-C<u>H</u>), 2.52 (3H, s, imidazole $CH_3$), 1.28 (3H, d, $C\underline{H}_3CH$).

Example 19b

(5R,6S)-3-[2-(2-Methyl-5-amino-imidazol-1-yl)ethylthio]-6-[(S)-1-hydroxyethyl]-7-oxo-1-azabicyclo[3.2.0]-hept-2-ene-2-carboxylic acid

The title compound (e91) was obtained in aqueous solution by hydrogenolysis of the ester (e90) (30 mg). The procedure outlined in Example 13c was followed with the exception that hydrogenolysis time was extended to 3 hours. A sample was freeze dried to yield a white fluffy solid, $\lambda_{max.}$ ($H_2O$) 297 nm, $\nu_{max.}$ (KBr) 3400, 1750, 1600, 1390 $cm^{-1}$.

Example 20

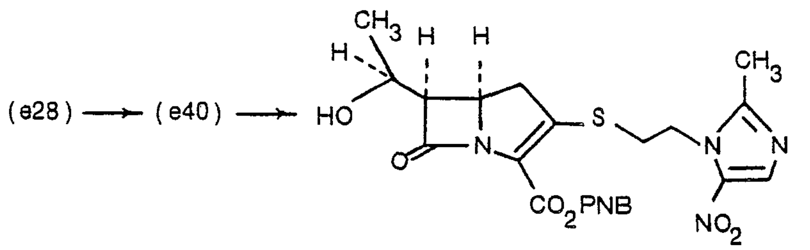


(e92)

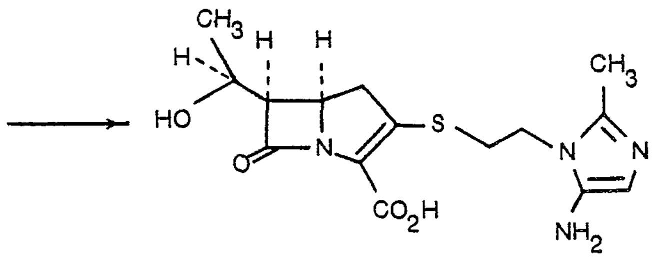

(e93)

Example 20a

p-Nitrobenzyl (5R,6R)-3-[2-(2-methyl-5-nitroimidazol-1-yl)ethylthio]-6-[(S)-1-hydroxyethyl]-7-oxo-1-azabicyclo[3.2.0]hept-2-ene-2-carboxylate

The title compound (e92) (77 mg) was prepared by reaction of the thiol (e40) derived from the ester (e28) (227 mg) with 1-(β-bromoethyl)-2-methyl-5-nitroimidazole, adopting the procedure described in Example 13., $\lambda_{max.}$ (EtOH) 310 nm (Em 17677), 266 nm (Em 12969), $\nu_{max.}$ (KBr) 3400, 1770, 1700, 1608, 1520, 740 $cm^{-1}$, $\delta_H$ ($CDCl_3$) 8.18 (2H, d, aromatic protons), 7.93 (1H, S, imidazole CH), 7.63 (2H, d, aromatic protons), 5.34 (2H, q, $CH_2Ar$), 4.51 (2H, t, $CH_2N$), 4.0—4.4 (m, 2H, 5-CH + 8-CH), 2.9—3.8 (5H, m, $SCH_2$ + 6-CH + 4-$CH_2$), 2.62 (1H, broad res., OH), 2.49 (3H, S, imidazole $CH_3$), 1.38 (3H, d, $CH_3CH$).

Example 20b

(5R,6R)-3-[2-(2-Methyl-5-aminoimidazol-1-yl)ethylthio]-6-[(S)-1-hydroxyethyl]-7-oxo-1-azabicyclo[3.2.0]-hept-2-ene-2-carboxylic acid

The title compound (e93) was prepared by hydrogenolysis of the ester (e92) (30 mg)], following the procedure outlined in Example 13c with the exception that the hydrogenation time was extended to 4 hours. Freeze drying gave a white solid, $\lambda_{max.}$ ($H_2O$) 293 nm, $\nu_{max.}$ (KBr) 3400, 1750, 1600, 1390 $cm^{-1}$.

Example 21

p-Nitrobenzyl (5R,6S)-3-[(2-p-nitrobenzyloxycarbonylaminothiazol-4-yl)methylthio]-6-[(S)-1-hydroxyethyl]-7-oxo-1-azabicyclo[3.2.0]hept-2-ene-2-carboxylate

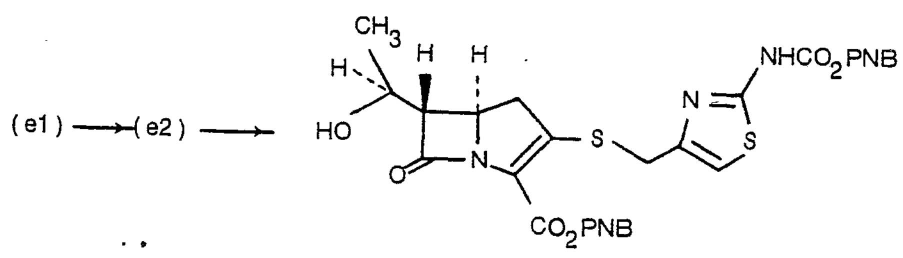


(e94)

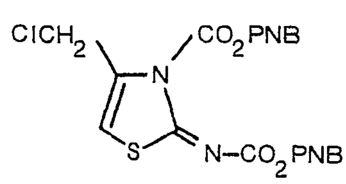

(e95)

Following the procedure described in Example 13, reaction of the thiol (e2) derived from the ester (e1) (250 mg) with the intermediate (e95) gave, after silica gel column chromatography, p-nitrobenzyl (5R,6S)-3-[(2-p-nitrobenzyloxycarbonylaminothiazol-4-yl)methylthio]-6-[(S)-1-hydroxyethyl]-7-oxo-1-azabicyclo-[3.2.0]hept-2-ene-2-carboxylate (e94) (70 mg) as a white solid, $\lambda_{max.}$ (EtOH) 319 nm (Em 12286), 263 nm (Em 25630), $\nu_{max.}$ (KBr) 3440, 1770, 1730, 1700, 1610, 1550, 1520, 1350, 1335 $cm^{-1}$, $\delta_H$ ($d_7$-DMF) 7.7—8.4 (8H, m, aromatic protons), 7.13 (1H, s, thiazole C<u>H</u>), 5.1—5.7 (s + q + broad d, 2 × $C\underline{H}_2Ar$ + O<u>H</u>, 5H), 4.0—4.4 (4H, s + m, $S\underline{CH}_2$ + 5-C<u>H</u> + 8-C<u>H</u>), 3.2—3.9 (m, 4-$C\underline{H}_2$ + 6-C<u>H</u>), 1.28 (3H, d, $C\underline{H}_3CH$).

Example 22

(e28) (e40)

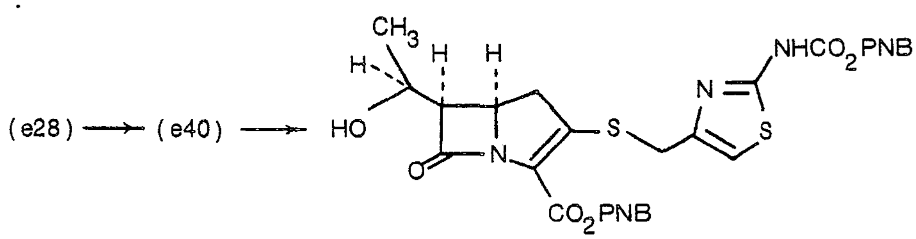

(e96)

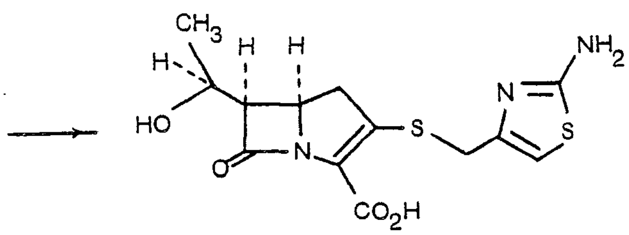

(e97)

Example 22a

p-Nitrobenzyl (5R,6R)-3-[2-p-nitrobenzyloxycarbonylaminothiazol-5-yl)methylthio]-6-[(S)-1-hydroxyethyl]-7-oxo-1-azabicyclo[3.2.0]hept-2-ene-2-carboxylate

The title compound (e96) (95 mg) was prepared as a white solid by reaction of the ester (e28) (250 mg) with N-bromoacetamide, followed by intermediate (e95), utilising the procedure outlined in Example 13., $\lambda_{max.}$ (EtOH) 316 nm (Em 13729), 264 nm (Em 27411), $\nu_{max.}$ (KBr) 3400 (broad), 3390 (sharp), 3080, 3110, 1770, 1730, 1695, 1608, 1550, 1345, 1330 $cm^{-1}$, $\delta_H$ ($d_7$-DMF) 7.7—8.4 (8H, m, aromatic protons), 7.15 (1H, s, thiazole-CH), 5.2—5.6 (4H, s + q, $C\underline{H}_2Ar$ protons), 4.0—4.4 (s + m, 4H, $S\underline{CH}_2$ + 5-C<u>H</u> + 8-C<u>H</u>), 1.32 (3H, d, $C\underline{H}_3CH$).

Example 22b

(5R,6R)-3-[(2-Aminothiazol-4-yl)methylthio]-6-[(S)-1-hydroxyethyl]-7-oxo-1-azabicyclo[3.2.0]hept-2-ene-2-carboxylic acid

The ester (e96) (60 mg) was hydrogenolysed, as in Example 13c to yield (5R,6R)-3-[(2-aminothiazol-4-yl)methylthio]-6-[(S)-1-hydroxyethyl]-7-oxo-1-azabicyclo[3.2.0]hept-2-ene-2-carboxylic acid (e97) (22 mg based on Em 8,500 at $\lambda_{max.}$ 297 nm in the u.v. spectrum) as an aqueous solution. A white fluffy solid was obtained on freeze-drying, $\lambda_{max.}$ ($H_2O$) 261 nm, 297 nm, $\nu_{max.}$ (KBr) 3400, 1750, 1590 $cm^{-1}$.

Example 23

p-Nitrobenzyl (5R,6S)-3-[2-phenylthiazol-4-yl)methylthio]-6-[(S)-1-hydroxyethyl]-7-oxo-1-azabicyclo[3.2.0]-hept-2-ene-2-carboxylate

(e1) (E2)

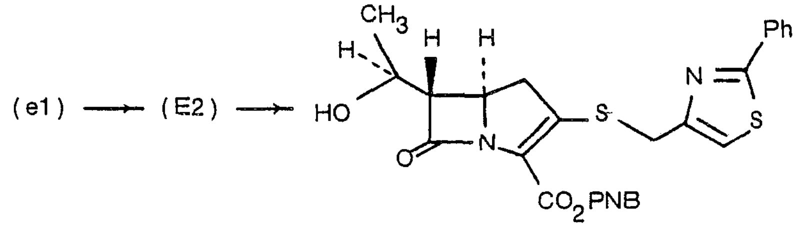

(e98)

p-Nitrobenzyl (5R,6S) - 3 - [(2 - phenylthiazol - 4 - yl)methylthio] - 6 - [(S) - 1 - hydroxyethyl] - 7 - oxo - 1 - azabicyclo[3.2.0]hept - 2 - ene - 2 - carboxylate (e98) (148 mg) was prepared as a pale yellow solid by reaction of the thiol (e2) derived from the ester (e1) (500 mg) with 2-phenyl-4-iodomethyl thiazole, following the procedure described in Example 13, $\lambda_{max.}$ (EtOH) 290 nm (Em 23059); $\nu_{max.}$ (KBr) 3500, 1768, 1698, 1608, 1546, 1519, 1348, 1332 $cm^{-1}$; $\delta_H$ ($d_7$-DMF) 7.4—8.4 (10H, m, p-nitrobenzyl protons + phenyl protons + thiazole-$C\underline{H}$), 5.44 (2H, q, $C\underline{H}_2Ar$), 5.19 (1H, d, $O\underline{H}$, disappears on $D_2O$), 4.42 (2H, s, $SC\underline{H}_2$), 4.0—4.4 (2H, m, 5-$C\underline{H}$ + 8-$C\underline{H}$), 3.5 (3H, m, 4-$C\underline{H}_2$ + 6-$C\underline{H}$), 1.29 (3H, d, $C\underline{H}_3CH$); m/e 537 (relative intensity) (1%), 491 (10), 451 (10), 286 (30), 207 (55), 175 (100), 136 (20).

Example 24

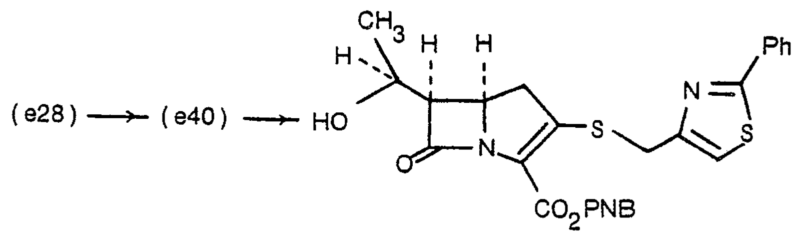


(e99)

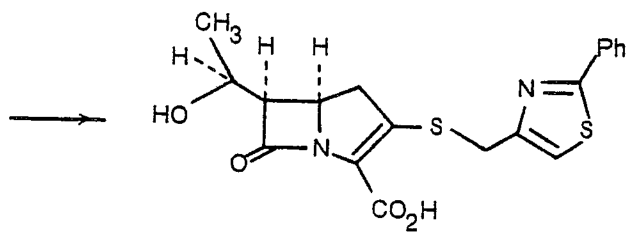

(e100)

Example 24a

p-Nitrobenzyl (5R,6R)-3-[(2-phenylthiazol-4-yl)methylthio]-6-[(S)-1-hydroxyethyl]-7-oxo-1-azabicyclo[3.2.0]-hept-2-ene-2-carboxylate

The title compound (e99) (198 mg) was obtained as a white solid by reaction of the thio (e40), derived from the ester (e28) (500 mg) with 2-phenyl-4-iodomethylthiazole, adopting the procedure described in Example 13, $\lambda_{max.}$ (EtOH) 300 nm (Em 21622); $\nu_{max.}$ (KBr) 3430, 3110, 1776, 1695, 1608, 1550, 1520, 1348, 1328 $cm^{-1}$; $\delta_H$ ($\delta_7$-DMF)7.4—8.3 (10H, m, p-nitrobenzyl protons + phenyl protons + thiazole-$C\underline{H}$), 5.43 (2H, q, $C\underline{H}_2Ar$), 5.10 (1H, d, $O\underline{H}$, disappears on $D_2O$), 4.47 (2H, s, $SC\underline{H}_2$), 3.9—4.4 (2H, m, 5-$C\underline{H}$ + 8-$C\underline{H}$), 3.5—3.9 (3H, m, 4-$C\underline{H}_2$ + 6-$C\underline{H}$), 1.33 (3H, d, $C\underline{H}_3CH$), m/e (relative intensity) 451 (4%), 418 (2), 286 (50), 207 (100), 174 (95), 104 (55), 71 (72).

Example 24b

(5R,6R)-3-[(2-Phenylthiazol-4-yl)methylthio]-6-[(S)-1-hydroxyethyl]-7-oxo-1-azabicyclo[3.2.0]hept-2-ene-2-carboxylic acid

The ester (e99) (90 mg) was hydrogenolysed, as in Example 13c, to yield the title compound (e100) (19 mg based on Em 17,000 at $\lambda_{max.}$ 297 nm in the u.v. spectrum) as an aqueous solution. A white fluffy solid was obtained on freeze-drying, $\lambda_{max.}$ ($H_2O$) 297 nm, $\nu_{max.}$ (KBr) 3400, 1740, 1600 $cm^{-1}$.

**Claims**

1. A process for the preparation of a compound of formula (I):

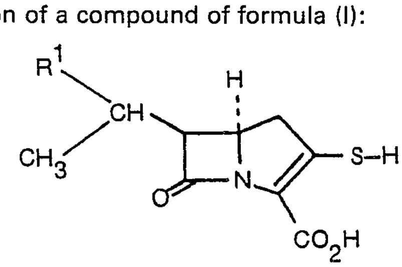

(I)

and salts and esters thereof,
wherein
the configuration at the 6-position may be R or S,

$R^1$ is hydrogen, OH, $OSO_3H$ or a salt or a $(C_{1-4})$alkyl ester thereof, $OR^2$, $SR^3$, $OCOR^2$, $OCO_2R^3$, or $OCONHR^3$,

$R^2$ is a $(C_{1-6})$alkyl group, a benzyl group, or a benzyl group substituted on the phenyl ring by $(C_{1-3})$alkoxy, fluorine, bromine, chlorine, or nitro, and

$R^3$ is a $(C_{1-6})$alkyl group, a benzyl group, a phenyl group, a benzyl group substituted on the phenyl ring by $(C_{1-3})$alkoxy, fluorine, bromine, chlorine, or nitro, or a phenyl group substituted by $(C_{1-3})$alkyl, $(C_{1-3})$alkoxy, fluorine, bromine, chlorine, or nitro, with the proviso that, when $R^1$ is $OSO_3H$ or a salt or $(C_{1-4})$alkyl ester thereof, the C-5 and C-6 hydrogen atoms are *cis;*

which process comprises the reaction of a cleavable ester of a compound of the formula (II):

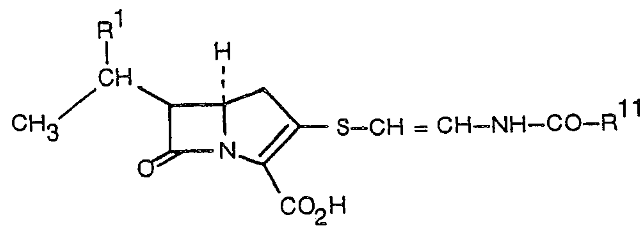

(II)

wherein

$R^{11}$ is a methyl or ethyl group;

with the proviso that, when $R^{11}$ is an ethyl group $R^1$ is $OSO_3H$ or a salt or $(C_{1-4})$alkyl ester thereof;

with a source of hypohalous acid, and optionally thereafter:

i) converting a cleavable ester to a free acid or salt,

ii) converting a cleavable ester to a different cleavable ester.

2. A process as claimed in claim 1 wherein the hypohalous acid is hypobromous acid.

3. A process as claimed in claim 2 wherein the source of hypobromous acid is N-bromoacetamide.

4. A compound of the formula (IA):

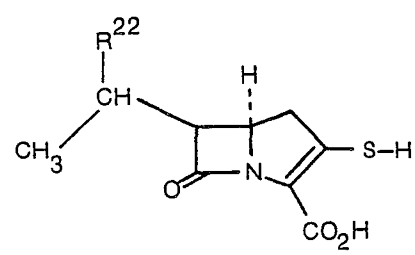

(IA)

and salts and esters thereof,

wherein

the configuration at the 6-position may be R or S,

$R^{22}$ is hydrogen, $OSO_3H$ or a salt or a $(C_{1-4})$alkyl ester thereof, $SR^3$ or $OCONHR^3$, and

$R^3$ is defined as in claim 1;

with the proviso that, when $R^{22}$ is $OSO_3H$ or a salt or $(C_{1-4})$alkyl ester thereof, the C-5 and C-6 hydrogen atoms are *cis.*

5. A compound as claimed in claim 4, in the form of a pharmaceutically acceptable salt or *in-vivo* hydrolysable ester.

6. A compound as claimed in either claim 4 or claim 5 wherein the C-6 substituent is 1-hydroxysulphonyloxyethyl or a pharmaceutically acceptable salt thereof.

7. A pharmaceutical composition which comprises a compound as claimed in any one of claims 4 to 6 and a pharmaceutically acceptable carrier.

8. A composition as claimed in claim 7, which also comprises a penicillin or cephalosporin.

**Patentansprüche**

1. Ein Verfahren zur Herstellung einer Verbindung der Formel (I):

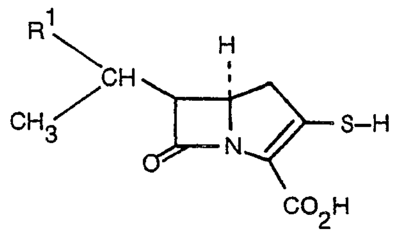

(I)

und Salzen und Estern derselben,
in welcher
die Konfiguration in der 6-Stellung R oder S sein kann, $R^1$ Wasserstoff, OH, $OSO_3H$ oder ein Salz oder $(C_{1-4})$Alkylester davon, $OR^2$, $SR^3$, $OCOR^2$, $OCO_2R^3$ oder $OCONHR^3$ darstellt,
$R^2$ eine $(C_{1-6})$Alkylgruppe, eine Benzylgruppe oder eine am Phenylring durch $(C_{1-3})$Alkoxy, Fluor, Brom, Chlor oder eine Nitrogruppe substituierte Benzylgruppe bedeutet, und
$R^3$ eine $(C_{1-6})$Alkylgruppe, eine Benzylgruppe, eine Phenylgruppe, eine am Phenylring durch $(C_{1-3})$Alkoxy, Fluor, Brom, Chlor oder eine Nitrogruppe substituierte Benzylgruppe oder eine Phenylgruppe substituiert durch $(C_{1-3})$Alkyl, $(C_{1-3})$Alkoxy, Fluor, Brom, Chlor oder eine Nitrogruppe, darstellt, mit der Maßgabe, daß wenn $R^1$ $OSO_3H$ oder ein Salz oder $(C_{1-4})$Alkylester davon ist, die C-5 und C-6-Wasserstoffatome in der cis-Stellung vorliegen;
welches Verfahren die Umsetzung eines spaltbaren Esters einer Verbindung der Formel (II):

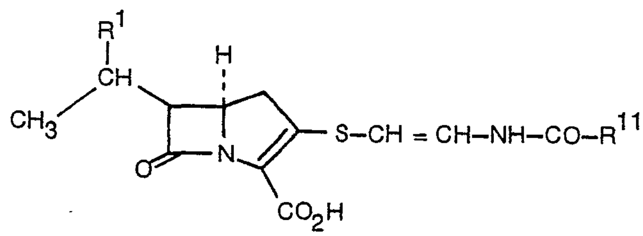

(II)

in welcher
$R^{11}$ eine Methyl- oder Äthylgruppe bedeutet,
mit der Maßgabe daß, wenn $R^{11}$ eine Äthylgruppe ist, $R^1$ $OSO_3H$ oder ein Salz oder $(C_{1-4})$Alkylester davon ist,
mit einer Quelle für unterhalogenige Säure, und gegebenenfalls anschließend
i) die Umwandlung eines spaltbaren Esters in eine freie Säure oder ein freies Salz,
ii) die Umwandlung eines spaltbaren Esters in einen anderen spaltbaren Ester,
umfaßt.

2. Ein Verfahren wie in Anspruch 1 beansprucht, in welchem die unterhalogenige Säure Hypobromsäure ist.

3. Ein Verfahren wie in Anspruch 2 beansprucht, in welchem die Quelle für Hypobromsäure N-Bromacetamid ist.

4. Eine Verbindung der formel (IA):

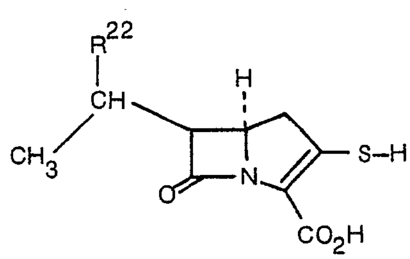

(IA)

und Salz und Ester derselben,
wobei
die Konfiguration in der 6-Stellung R oder S ist,
$R^{22}$ Wasserstoff, $OSO_3H$ oder ein Salz oder $(C_{1-4})$Alkylester davon, $SR^3$ oder $OCONHR^3$ ist, und
$R^3$ wie in Anspruch 1 definiert ist,
mit der Maßgabe, daß wenn $R^{22}$ $OSO_3H$ oder ein Salz oder $(C_{1-4})$Alkylester davon ist, die C-5 und C-6-Wasserstoffatome sich in der cis-Stellung befinden.

5. Eine Verbindung wie in Anspruch 4 beansprucht, in Form eines pharmazeutisch verträglichen Salzes oder eines in-vivo hydrolysierbaren Esters.

6. Eine Verbindung wie in einem der beiden Ansprüche 4 oder 5 beansprucht, in welcher der am C-Atom in der 6-Stellung befindliche Substituent 1-Hydroxysulphonyloxyäthyl ist, oder ein pharmazeutisch verträgliches Salz davon.

7. Eine pharmazeutische Zusammensetzung, welche eine Verbindung wie in einem der Ansprüche 4 bis 6 beansprucht und einen pharmazeutisch verträglichen Träger umfaßt.

8. Eine Zusammensetzung wie in Anspruch 7 beansprucht, welche auch ein Penicillin oder Cephalosporin umfaßt.

**Revendications**

1. Procédé de préparation d'un composé de formule

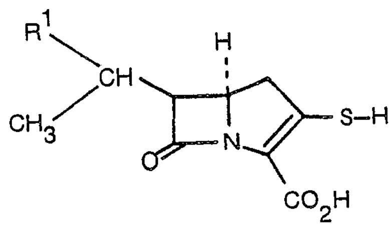

(I)

de ses sels et esters, dans laquelle:

la configuration en position 6 peut être R ou S,

$R^1$ est un atome d'hydrogène, un radical OH, $OSO_3H$ ou un sel ou un ester alcoylique de celui-ci, $OR^2$, $SR^3$, $OCOR^2$, $OCO_2R^3$ ou $OCONHR^3$.

$R^2$ est un groupe alcoyle en $C_{1-6}$, benzyle, ou zyle dont le cycle phényle est substitué par un groupe alcoxy en $C_{1-3}$, ou nitro ou un atome de fluor, brome ou chlore, et

$R^3$ est un groupe alcoyle en $C_{1-6}$, un groupe benzyle, un groupe phényle, un groupe benzyle dont le cycle phényle est substitué par un groupe alcoxy en $C_{1-3}$, nitro ou un atome de fluor, brome ou chlore, ou un groupe phényle substitué par un groupe alcoyle en $C_{1-3}$, alcoxy en $C_{1-3}$, nitro ou un atome de fluor, brome ou chlore,

à condition que dans le cas où $R^1$ est $OSO_3H$ ou un sel ou un ester alcoylique de celui-ci, les atomes d'hydrogène en C-5 et C-6 soient en position cis;

lequel procédé comprend la réaction d'un ester détachable d'un composé de formule (II)

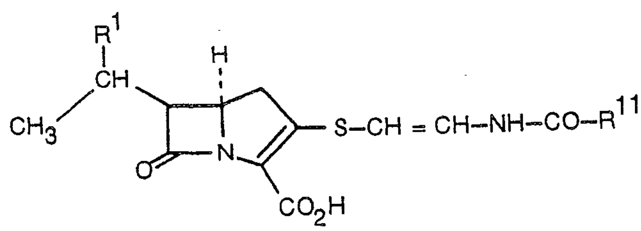

(II)

dans laquelle

$R^{11}$ est un groupe méthyle ou éthyle;

à condition que, dans le cas où $R^{11}$ est un groupe éthyle, $R^1$ soit $OSO_3H$ ou un sel ou un ester alcoylique en $C_{1-4}$ de celui-ci;

avec une source d'acide hypohalogéneux et éventuellement ensuite:

i) la conversion d'un ester détachable en un acide libre ou un sel,

ii) la conversion d'un ester détachable en un ester détachable différent.

2. Procédé suivant la revendication 1, caractérisé en ce que l'acide hypohalogéneux est l'acide hypobromeux.

3. Procédé suivant la revendication 2, caractérisé en ce que la source d'acide hypotenseur est le N-bromoacétamide.

4. Composé de formule (IA)

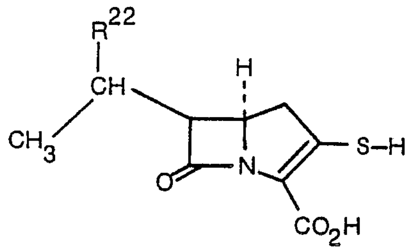

(IA)

ses sels, et ses esters, dans laquelle

la configuration en position 6 peut être R ou S,

$R^{22}$ est un atome d'hydrogène, $OSO_3H$ ou un sel ou un ester alcoylique en $C_{1-4}$ de celui-ci; $SR^3$ ou $OCONHR^3$, et

$R^3$ est tel que défini dans la revendication 1;

à condition que, dans le cas où $R^{22}$ est $OSO_3H$ ou un sel ou un ester alcoylique en $C_{1-4}$ de celui-ci, les atomes d'hydrogène en C-5 et C-6 soient en position cis.

5. Composé suivant la revendication 4, sous la forme d'un sel acceptable du point de vue pharmaceutique ou d'un ester hydrolysable in vivo.

6. Composé suivant la revendication 4 ou la revendication 5, caractérisé en ce que le substituant porté en C-6 est un groupe 1-hydroxysulfonyloxyéthyle ou un sel acceptable du point de vue pharmaceutique de celui-ci.

7. Composition pharmaceutique, caractérisée en ce qu'elle comprend un composé suivant l'une quelconque des revendications 4 à 6 et un support acceptable du point de vue pharmaceutique.

8. Composition suivant la revendication 7, qui comprend également une pénicilline ou une céphalosporine.